# EUROPÄISCHE PATENTSCHRIFT

(11) **EP 0 517 660 B1**
(45) Veröffentlichungstag und Bekanntmachung des Hinweises auf die Patenterteilung: **23.04.1997**
(21) Anmeldenummer: 92810403.3
(22) Anmeldetag: 26.05.1992
(51) Int. Cl.: C07D 285/14, A01N 43/82

(54) **Benzo-1,2,3-thiadiazol-Derivate**
Benzo-1,2,3-thiadiazole derivatives
Dérivés de benzo-1,2,3-thiadiazole

(30) Priorität: 05.06.1991 CH 1668/91
(43) Veröffentlichungstag der Anmeldung: 09.12.1992
(73) Patentinhaber: Novartis AG, 4058 Basel (CH)
(72) Erfinder: Brunner, Hans-Georg, Dr., CH-4415 Lausen (CH); Kunz, Walter, Dr., CH-4104 Oberwil (CH); Schurter, Rolf, Dr., CH-4102 Binningen (CH)

(56) Entgegenhaltungen:
- EP-A- 0 313 512
- EP-A- 0 384 889
- EP-A- 0 384 890
- EP-A- 0 387 195
- DE-A- 4 005 175
- GB-A- 1 177 972
- US-A- 4 946 981

## Beschreibung

Die vorliegende Erfindung betrifft neue substituierte Benzo-1,2,3-thiadiazol-Derivate der nachstehenden Formel I. Die Erfindung betrifft ferner die Herstellung dieser Substanzen sowie die als Wirkstoffe mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, insbesondere pflanzenschädigende Pilze.

Die erfindungsgemässen Verbindungen entsprechen der allgemeinen Formel I 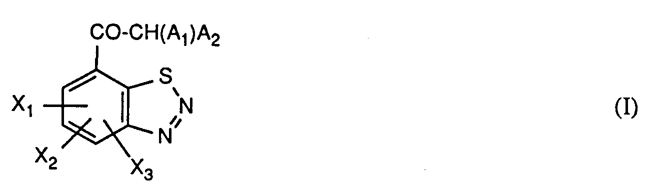 in welcher bedeuten:
A₁ und A₂ unabhängig voneinander CO-C₁-C₄-Alkyl, COO-C₁-C₄-Alkyl, CO-CF₃, CON(R)₂ oder Cyano;
A₁ und A₂ zusammen CO(X)ₙ-C₁-C₃-Alkylen-(X)ₙCO, durch C₁-C₄-Alkyl, COOR, CON(R)₂, Cyano oder Phenyl substituiertes CO(X)ₙ-C₁-C₃-Alkylen-(X)ₙCO, wobei der Phenylring seinerseits durch Halogen, Methyl, Trifluormethyl, Methoxy, Nitro oder Cyano substituiert sein kann; CO-N(R)-CO-N(R)-CO;
R Wasserstoff, C₁-C₆-Alkyl, C₃-C₆-Alkenyl oder C₃-C₆-Alkinyl;
X Sauerstoff, Schwefel oder N(CH₃);
X₁, X₂, X₃ unabhnängig voneinander Wasserstoff, Halogen, Methyl, Methylthio, Methoxy oder Nitro;
n 0 oder 1;
unter Einschluss der Salze der Verbindungen der Formel I mit pflanzenverträglichen organischen oder anorganischen Basen sowie der Metallkomplexe.

Halogen bedeutet Fluor, Chlor, Brom oder Jod, bevorzugt Fluor und weiter in der Reihenfolge Chlor, Brom und Jod. Als Substituent in einzelnen Resten kann Halogen bis zu 3-fach vertreten sein.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende bevorzugte Gruppen dar: Methyl, Ethyl sowie die Isomeren von Propyl oder Butyl, wie z.B. Isopropyl, Isobutyl, tert.-Butyl oder sek.-Butyl.

Alkenyl steht z.B. für Propenyl-(1), Allyl, Butenyl-(1), Butenyl-(2) oder Butenyl-(3) und Alkinyl bedeutet z.B. Propinyl-(2), Butinyl-(1), Pentinyl-(4) oder Hexinyl-(2).

Die Erfindung betrifft sowohl die freien Verbindungen der Formel I, als auch deren Additionssalze mit anorganischen und organischen Basen, ebenso deren Komplexe mit Metallsalzen.

Erfindungsgemässe Salze sind insbesondere Salze mit nach Massgabe des Einsatzzweckes physiologisch unbedenklichen anorganischen oder organischen Basen, wie beispielsweise Alkali- oder Erdalkalihydroxyde oder -oxide oder -Carbonate, -Hydrogencarbonate oder Amine wie mono-, di- oder trialkylamine oder Heterocyclische Basen wie Pyridinbasen (z.B. Pyridin, 4-Dimethylaminopyridin, Collidin), oder auch Additionssalze mit geeigneten Salzen, wie beispielsweise Magnesium- oder Calciumchlorid.

Metallkomplexe der Formel I bestehen aus dem zugrundeliegenden organischen Molekül und einem anorganischen oder organischen Metallsalz, z.B. den Halogeniden, Nitraten, Sulfaten, Phosphaten, Tartraten usw. z.B. des Magnesiums, Calciums, Bariums, Zinns, Kupfers, Mangans, Eisens, Zinks und Nickels sowie weiterer Metalle. Dabei können die Metallkationen in den verschiedenen ihnen zukommenden Wertigkeiten vorliegen.

Aufgrund ihrer besonderen pflanzenschützenden Eigenschaften lassen sich die Wirkstoffe der Formel I in folgende Gruppen einteilen:
a) Verbindungen der Formel I, worin bedeuten:
   A₁ und A₂ zusammen CO(X)ₙ-C₁-C₃-Alkylen-(X)ₙCO, durch C₁-C₄-Alkyl, COOH, COOCH₃, COOC₂H₅, COOCH₂-CH=CH₂, COOCH₂-C≡CH, Phenyl, die Gruppe CO-N(CH₃)₂ oder Cyano substituiertes CO-C₁-C₃-Alkylen-CO; CO-N(R′)-CO-N(R′)-CO;
   X Sauerstoff oder Schwefel;
   R′ Wasserstoff, C₁-C₃-Alkyl;
   X₁, X₂, X₃ unabhängig voneinander Wasserstoff, F, Methyl, Methoxy, Methylthio oder Nitro;
   n 0 oder 1;
   unter Einschluss der Salze der Verbindungen der Formel I mit organischen oder anorganischen Basen sowie der Metallkomplexe mit Mg, Ca, Ba, Cu, Sn, Fe, Zn, Cu, Ni oder Mn.
b) Verbindungen der Formel I, worin bedeuten:
   A₁ und A₂ unabhängig voneinander CO-C₁-C₄-Alkyl, COO-C₁-C₄-Alkyl, CO-CF₃, CO-N(R)₂ oder Cyano;
   R Wasserstoff, C₁-C₆-Alkyl, C₃-C₆-Alkenyl oder C₃-C₆-Alkinyl;
   X₁, X₂, X₃ unabhängig voneinander Wasserstoff, Halogen, Methyl, Methylthio, Methoxy oder Nitro;
   unter Einschluss der Salze der Verbindungen der Formel I mit pflanzenverträglichen organischen oder anorganischen Basen sowie der Metallkomplexe mit Mg, Ca, Ba, Cu, Sn, Fe, Zn, Ni oder Mn.
a₁) Verbindungen der Formel I, worin bedeuten:
   A₁ und A₂ zusammen CO(X)ₙ-C₁-C₃-Alkylen-(X)ₙCO, durch C₁-C₄-Alkyl, COOH, COOCH₃, Phenyl oder die Gruppe CO-N(CH₃)₂ substituiertes CO(X)ₙ-C₁-C₃-Alkylen-(X)ₙCO;
   X Sauerstoff;
   X₁, X₂, X₃ unabhängig voneinander Wasserstoff, Fluor oder Methyl; n 0 oder 1.
b₁) Verbindungen der Formel I, worin bedeuten:
   A₁ und A₂ unabhängig voneinander CO-C₁-C₂-Alkyl, COO-C₁-C₂-Alkyl, CO-CF₃ oder CO-N(R)₂;
   X₁, X₂, X₃ unabhängig voneinander Wasserstoff, Fluor oder Methyl.
a₂) Verbindungen der Formel I, worin bedeuten:
   A₁ und A₂ zusammen CO-C₁-C₃-Alkylen-CO, durch C₁-C₂-Alkyl, COOH oder Phenyl substituiertes CO-C₁-C₃-Alkylen-CO;
   X₁, X₂, X₃ unabhängig voneinander Wasserstoff oder Fluor.
b₂) Verbindungen der Formel I, worin bedeuten:
   A₁ und A₂ unabhängig voneinander CO-C₁-C₂-Alkyl, COO-C₁-C₂-Alkyl oder CO-N(CH₃)₂;
   X₁, X₂, X₃ unabhängig voneinander Wasserstoff oder Fluor.
a₃) Verbindungen der Formel I, worin bedeuten:
   A₁ und A₂ zusammen CO-C₁-C₃-Alkylen-CO, durch Methyl substituiertes CO-C₁-C₃-Alkylen-CO;
   X₁, X₂, X₃ unabhängig voneinander Wasserstoff oder Fluor.
b₃) Verbindungen der Formel I, worin bedeuten:
   A₁ und A₂ unabhängig voneinander CO-CH₃, COOC₁-C₄-Alkyl, CN oder CON(CH₂C≡CH)₂;
   X₁, X₂, X₃ unabhängig voneinander Wasserstoff oder Fluor.
a₄) Verbindungen der Formel I, worin bedeuten:
   A₁ und A₂ zusammen CO-N(R′)-CO-N(R′)-CO;
   R′ Wasserstoff, C₁-C₃-Alkyl;
   X₁, X₂, X₃ unabhängig voneinander Wasserstoff, Fluor oder Methyl.
a₅) Verbindungen der Formel I, worin bedeuten:
   A₁ und A₂ zusammen CO-N(CH₃)-CO-N(CH₃)-CO;
   X₁, X₂, X₃ unabhängig voneinander Wasserstoff oder Fluor.

Folgende Wirkstoffe der Formel I zeichnen sich durch besonders vorteilhafte pflanzenschützende Eigenschaften aus:
2-(Benzo-1,2,3-thiadiazol-7-carbonyl)-3-hydroxy-cyclohex-2-enon;
2-(Benzo-1,2,3-thiadiazol-7-carbonyl)-3-hydroxy-5,5-dimethyl-cyclohex-2-enon;
2-(Benzo-1,2,3-thiadiazol-7-carbonyl)-3-hydroxy-5-methyl-cyclohex-2-enon;
2-(Benzo-1,2,3-thiadiazol-7-carbonyl)-3-hydroxy-5-isopropyl-cyclohex-2-enon;
2-(Benzo-1,2,3-thiadiazol-7-carbonyl)-3-hydroxy-5-phenyl-cyclohex-2-enon;
2-(6-Fluor-benzo-1,2,3-thiadiazol-7-carbonyl)-3-hydroxy-5,5-dimethyl-cyclohex-2-enon;
2-(4-Fluor-benzo-1,2,3-thiadiazol-7-carbonyl)-3-hydroxy-cyclohex-2-enon;
5-(Benzo-1,2,3-thiadiazol-7-carbonyl)-1,3-dimethyl-2,4,6-1H,3H,5H-pyrimidintrion; (Benzo-1,2,3-thiadiazol-7-carbonyl)-malonsäurediethylester.

Die Verbindungen der Formel I werden wie folgt hergestellt:
a) durch Acylierung von Methylenverbindungen der Formel II

   A₁-CH₂-A₂ (II)

   mit einem aktivierten Säurederivat der Formel III 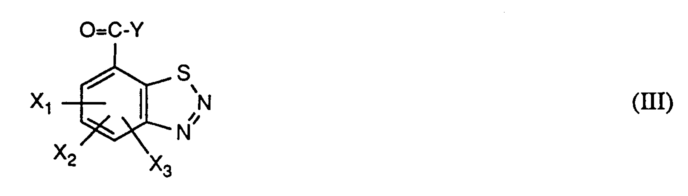 in Gegenwart einer Base mit oder ohne Zusatz von Metallsalzen, wie MgCl₂ oder MgO, oder einer Lewis-Säure in inerten Lösungsmitteln bei Temperaturen von -30°C bis 180°C, vorzugsweise 0° bis 130°C; wobei Y Halogen, Cyano oder den Rest 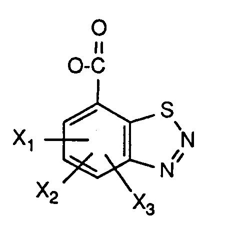 darstellt und A₁, A₂, X₁, X₂ und X₃ die unter Formel I angegebenen Bedeutungen besitzen; oder
b) aus Methylenverbindungen der Formel IIa 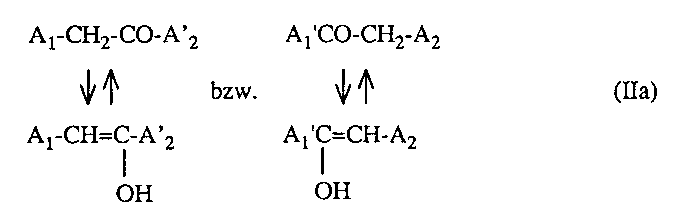 durch Acylierung der Enolform am Sauerstoffatom mit einer Verbindung der Formel III 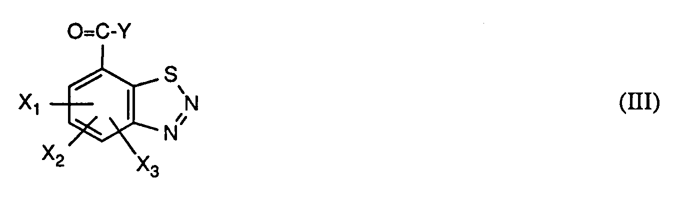 in Gegenwart einer Base in einem inerten Lösungsmittel bei Temperaturen von - 30°C bis 160°C über die Zwischenprodukte der Formel IV 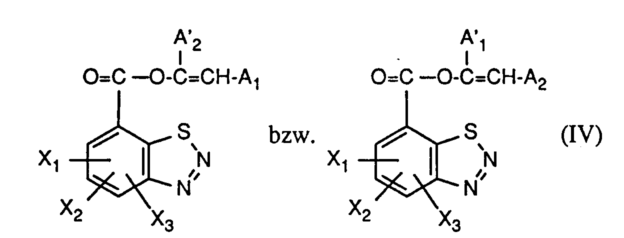 wobei A₁′ bzw. A₂′ folgende Bedeutungen hat:
   C₁-C₄-Alkyl, O-C₁-C₄-Alkyl, CF₃, N(R)₂, unsubstituiertes oder durch C₁-C₄-Alkyl, COOR, CON(R)₂, Cyano oder Phenyl substituiertes (X)ₙ-C₁-C₃-Alkylen-(X)ₙCO, wobei der Phenylring seinerseits durch Halogen, Methyl, Trifluormethyl, Methoxy, Nitro oder Cyano substituiert sein kann; und N(R)-CO-N(R)-CO;
   und diese mit einer Base mit oder ohne Zusatz von Cyanid-Verbindungen oder einer Lewis-Säure, wie z.B. AlCl₃, als Katalysator in inerten Lösungsmitteln bei Temperaturen von -30°C bis 160°C, bevorzugt 0° bis 120°C, in Verbindungen der Formel Ia 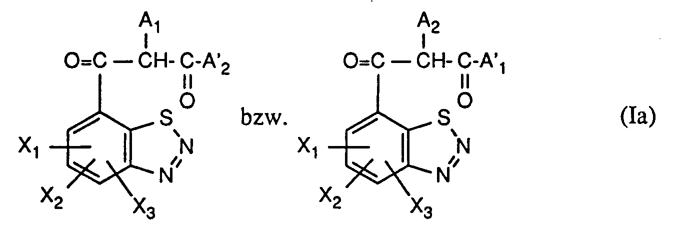 umlagert, wobei die Verbindungen der Formel I und Ia auch in den Enolformen der Formeln Ia′ 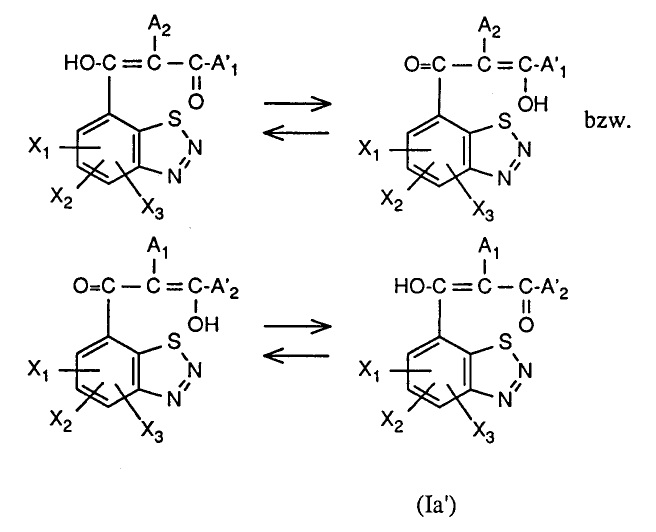 vorliegen können; und wobei X₁, X₂, X₃, A₁, A₂, A₁′ und A₂′ die unter Formel I, II und IV angegebenen Bedeutungen besitzen und A₁ und A′₂ bzw. A′₁ und A₂ zusammen C₁-C₃-Alkylen-(X)ₙCO, durch C₁-C₄-Alkyl-COOR, COOR, CON(R₂), Cyano oder Phenyl substituiertes C₁-C₃-Alkylen-(X)ₙCO oder N(R)-CO-N(R)-CO darstellt, wobei R Wasserstoff, C₁-C₆-Alkyl, C₃-C₆-Alkenyl oder C₃-C₆-Alkinyl bedeutet.

Zur Verwendung in den vorstehend beschriebenen Verfahren kommen folgende inerte Lösungsmittel in Frage: aliphatische, cycloaliphatische oder aromatische Kohlenwasserstoffe, wie z.B. Hexan, Cyclohexan, Toluol, Xylol, Petrolether oder Ligroin; chlorierte Kohlenwasserstoffe, wie z.B. Methylenchlorid, Chloroform, Tetrachlorkohlenstoff, Chlorbenzol; Ether, wie z.B. Diethylether, Diisopropylether, Furan, Tetrahydrofuran, Dioxan; Ketone, wie z.B. Aceton, Methylethylketon; Alkohole, wie z.B. Methanol, Ethanol, Isopropanol; Ester, wie z.B. Essigsäureethylester, Essigsäurebutylester; Nitrile wie z.B. Acetonitril, Propionitril; Säureamide, wie z.B. Dimethylformamid; Sulfone und Sulfoxide, wie z.B. Dimethylsulfoxid, Sulfolan.

Als Basen oder säurebindende Mittel eignen sich z.B. Hydroxide, Carbonate, Hydrogencarbonate oder Alkoholate der Alkalimetalle; sowie tertiäre Amine, wie z.B. Trialkylamin, Pyridin oder 4-N,N-Dialkylaminopyridin.

Ebenfalls finden in den beschriebenen Verfahren Lewis-Säuren Anwendung, wie z.B. die Halogenide des Bors, Aluminiums, Magnesiums, Zinks, Antimons, Quecksilbers, Kupfers oder Silbers.

Durch Zusätze von Metallsalzen, z.B. mit den Kationen Mg²⁺, Ca²⁺, Ba²⁺, Cu²⁺, Ni²⁺, Fe²⁺/Fe³⁺, Zn²⁺, Sn²⁺ oder Mn²⁺, oder ferner von anorganischen oder organischen Basen können aus den Verbindungen der Formel I und Ia entsprechende Salze oder Komplexe erhalten werden. Sie stellen soweit sie pflanzenverträglich sind ebenfalls einen Teil der Erfindung dar.

Die vorstehend beschriebenen Verfahren entsprechen Synthesemethoden, die aus der Literatur bekannt sind. Sie sind beispielsweise in der U.S. Patentschrift Nr. 4,946,981 beschrieben.

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mikroorganismen mittels Blattapplikation oder Bodenapplikation als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der besonderen Wirkungsweise der erfindungsgemässen Verbindungen der Formel I, nämlich einerseits der Möglichkeit einer direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I eigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. Hemileia, Rhizocotonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden:
Pilze, wie z.B. Oomyceten (z.B. Plasmopara viticola, Phytophthora infestans, Peronospora tabacina, Pseudoperonospora, Bremia letucae), Fungi imperfecti (z.B. Colletotrichum lagenarium, Pyricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaik-virus.

Die erfindungsgemässen Verbindungen können zum Schutz von Pflanzen unterschiedlicher Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen).

Diese Aufzählung stellt keine Limitierung dar.

Besonders geeignete Zielkulturen für die Anwendung der erfindungsgemässen Verbindungen der Formel I sind folgende Pflanzen: Gurke, Tabak, Reben, Reis, Pfeffer, Kartoffeln, Tomaten, Weizen, Gerste, Birnen und Aepfel.

Als besonderen Vorteil der erfindungsgemässen Verbindungen der Formel I ist neben ihren zielgerichteten Aktivitäten im Pflanzenschutz das Fehlen von phytotoxischen Eigenschaften anzusehen.

Die im Rahmen der Erfindung zur Anwendung gelangenden mikrobiziden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (C₁₀-C₂₂), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

### Beispiel 1.1: Herstellung von 3-(Benzo-1,2,3-thiadiazol-7-carbonyloxy)-5,5-dimethyl-cyclohex-2-enon (Zwischenprodukt)

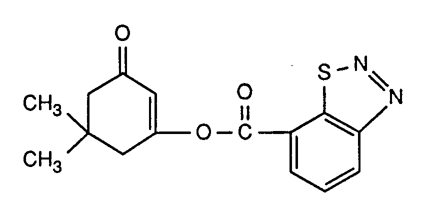

5.6 g (0,04 Mol) 5,5-Dimethyl-1,3-cyclohexandion werden in einem Gemisch von 4,4 g (0,044 Mol) Triethylamin und 120 ml Essigsäureethylester gelöst. Bei 15° bis 20°C werden 8,7 g (0,044 Mol) Benzthiadiazol-7-carbonsäurechlorid zugetropft und anschliessend über Nacht bei Raumtemperatur gerührt. Die Essigsäureesterlösung wird mit H₂O gewaschen, getrocknet und eingeengt. Säulenchromatographische Reinigung mit Essigsäureethylester/Hexan ergibt 10,5 g (87,5 %) der Titelverbindung mit einem Schmelzpunkt von 131-133°C.

| Elementaranalyse | | | | |
|---|---|---|---|---|
| | C | H | N | S |
| gef. | 59,8 | 4,7 | 9,2 | 10,6 |
| ber. | 59,59 | 4,67 | 9,27 | 10,61 |

### Beispiel 1.2: Herstellung von 2-(Benzo-1,2,3-thiadiazol-7-carbonyl)-5,5-dimethyl-1,3-cyclohexandion

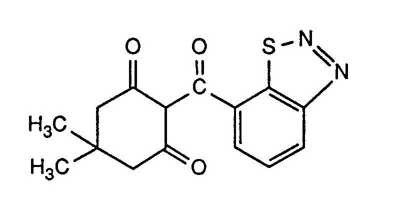

8,5 g 3-(Benzo-1,2,3-thiadiazol-7-carbonyloxy)-5,5-dimethyl-cyclohex-2-enon (Beispiel 1.1), 0,09 g KCN und 2,8 g Triethylamin werden in 40 ml Dimethylformamid gelöst und über Nacht bei Raumtemperatur gerührt. Die Reaktionslösung wird auf Eiswasser gegossen, mit conc. HCl angesäuert und das Produkt abgesaugt. Es werden 7,7 g (90,6 %) der Titelverbindung mit einem Schmelzpunkt von 168-170°C erhalten.

| Elementaranalyse | | | | |
|---|---|---|---|---|
| | C | H | N | S |
| gef. | 59,44 | 4,84 | 9,42 | 10,56 |
| ber. | 59,59 | 4,67 | 9,27 | 10,61 |

### Beispiel 1.3: Herstellung von-5-(Benzo-1,2,3-thiadiazol-7-yl-2,2-dimethyl-1,3-dioxan-4,6-dion (2:1 Salz mit 4-Dimethylaminopyridin)

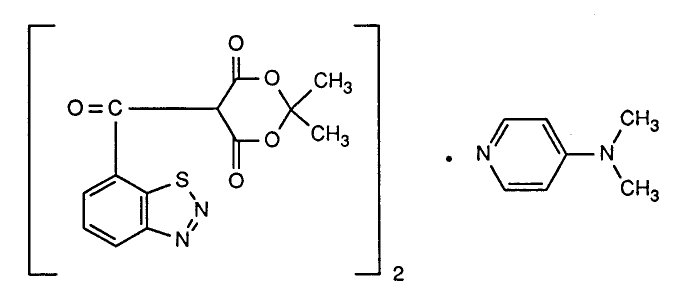

5,5 g Benzo-1,2,3-thiadiazol-7-carbonsäurechlorid in 10 ml Tetrahydrofuran werden zu einer bei 0°C gerührten Lösung von 3,6 g Malonsäure-cycl.-isopropylidenester, 7,6 ml Triethylamin und 2.0 g 4-Dimethylaminopyridin in 25 ml Dichlormethan zugetropft. Nach 2 Std. Rühren bei 0°C wird das Reaktionsgemisch direkt auf eine Kieselgelsäule gegeben und mit Essigsäureethylester extrahiert, wobei die Titelverbindung als Salz mit 4-Dimethylaminopyridin (2:1) vom Smp. 170-172°C resultiert.

### Beispiel 1.4: Herstellung von2-(Benz-1,2,3-thiadiazol-7-yl)-malonsäurediethylester (Verb. Nr. 4.15)

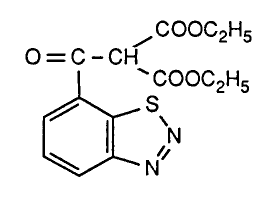

4,8 g Magnesiumchlorid in 50 ml Acetonitril werden unter Kühlen mit Eis und Rühren mit 7,6 ml Malonsäurediethylester und 14 ml Triethylamin versetzt. Nach Rühren während 20 Min. bei 0-5°C wird portionenweise mit einer Suspension von 9,6 g Benz-1,2,3-thiadiazol-7-carbonsäurechlorid in 50 ml Acetonitril versetzt, wobei die Temperatur zwischen 5°C und 15°C gehalten wird. Die erhaltene beige Suspension wird während 1 Stunde weiter im Eisbad bei 0°C und anschliessend über Nacht bei Raumtemperatur gerührt. Anderntags wird wieder auf 0°C gekühlt, mit 15%iger Salzsäure auf pH 4,5 angesäuert und mit Essigsäureethylester extrahiert. Die Extrakte werden über Natriumsulfat getrocknet, über Kieselgel filtriert und eingedampft. Dabei resultiert die Titelverbindung in einer Ausbeute von 83,2 % d.Th. und einem Schmelzpunkt von 72-74°C.

Die Verbindungen der Tabellen 1-7 werden analog hergestellt.

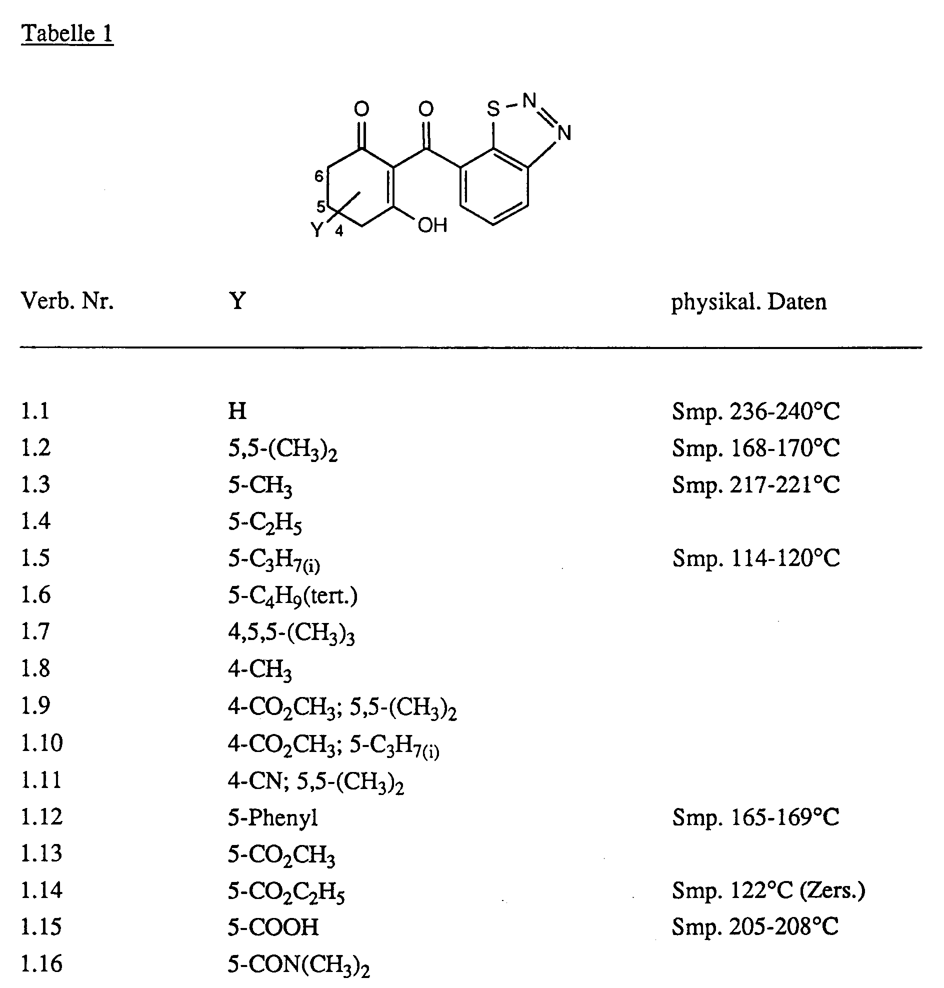

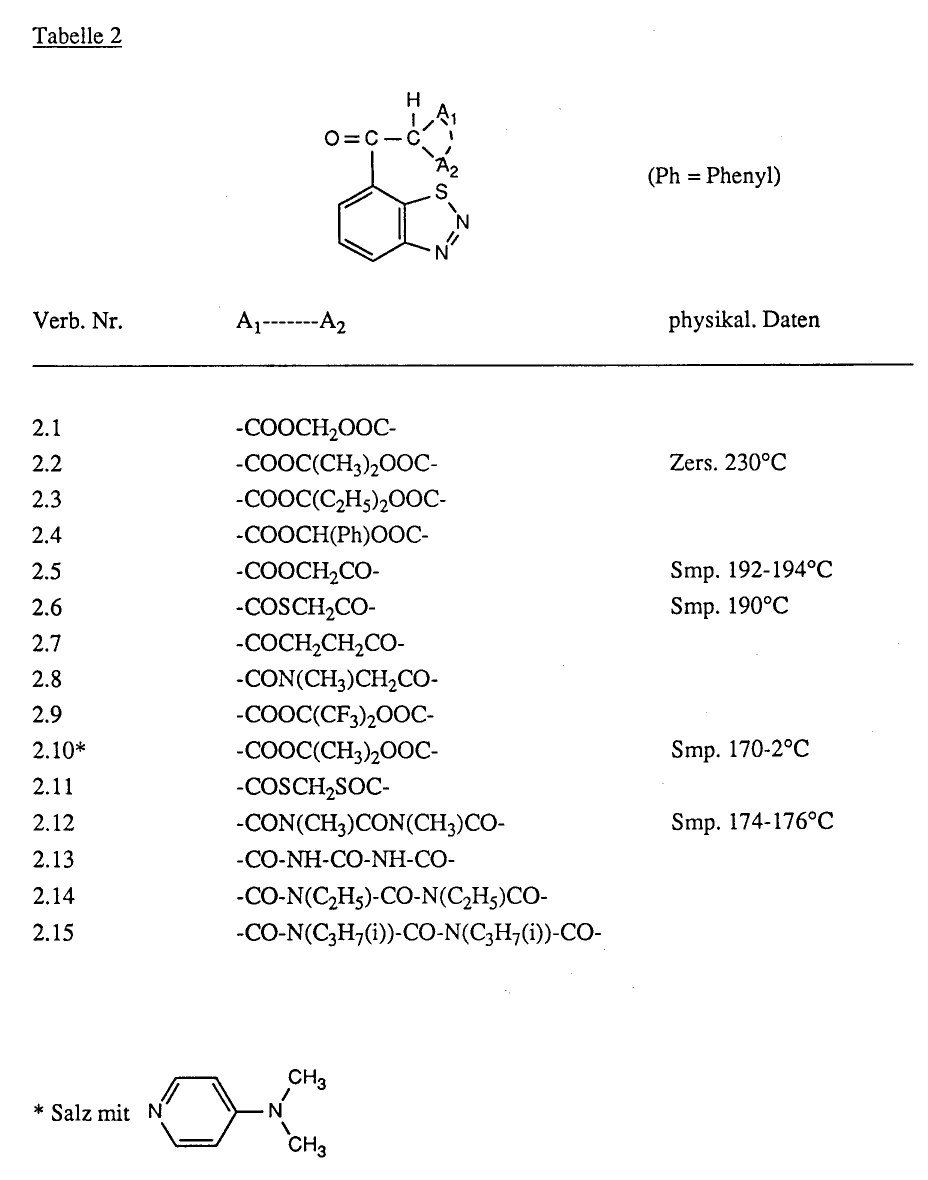

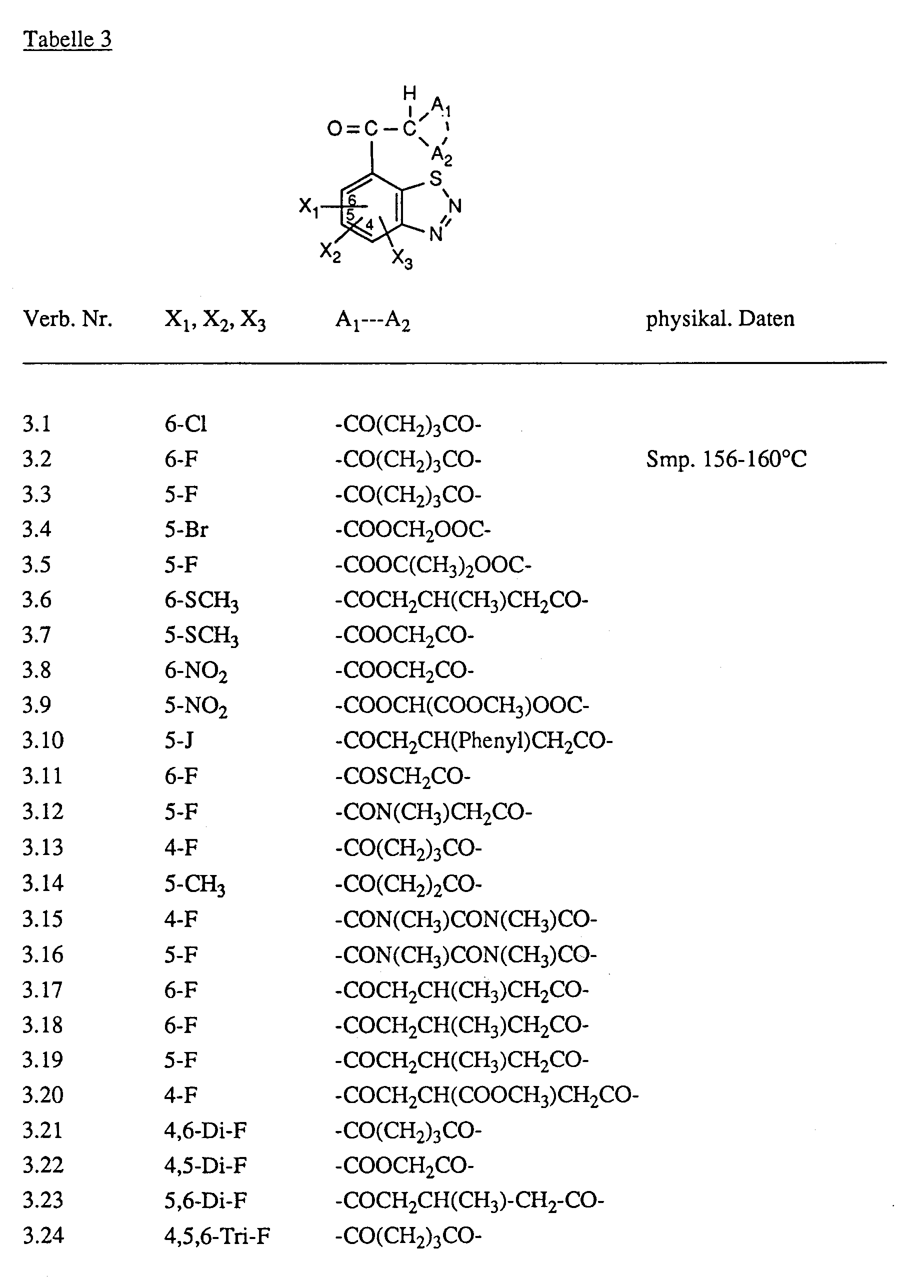

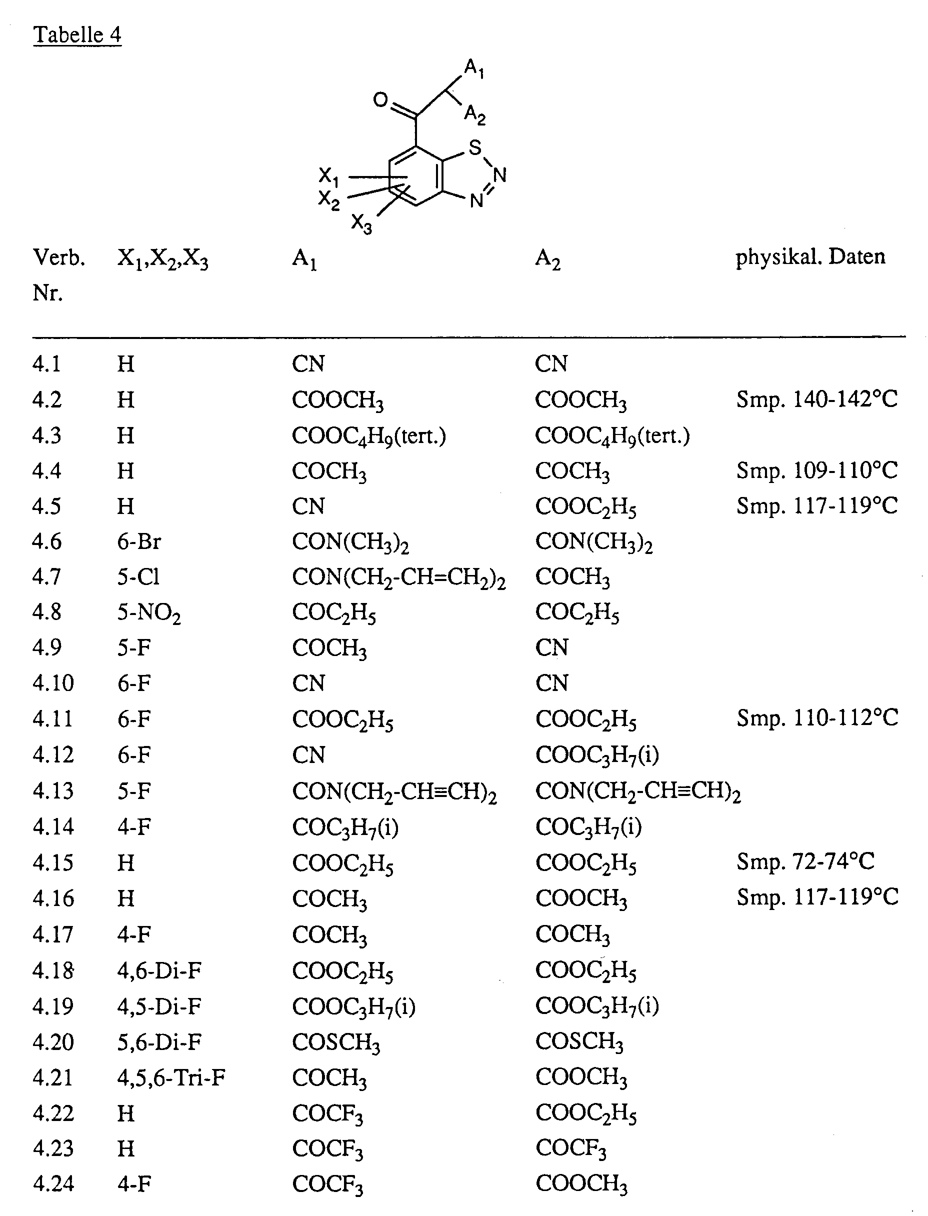

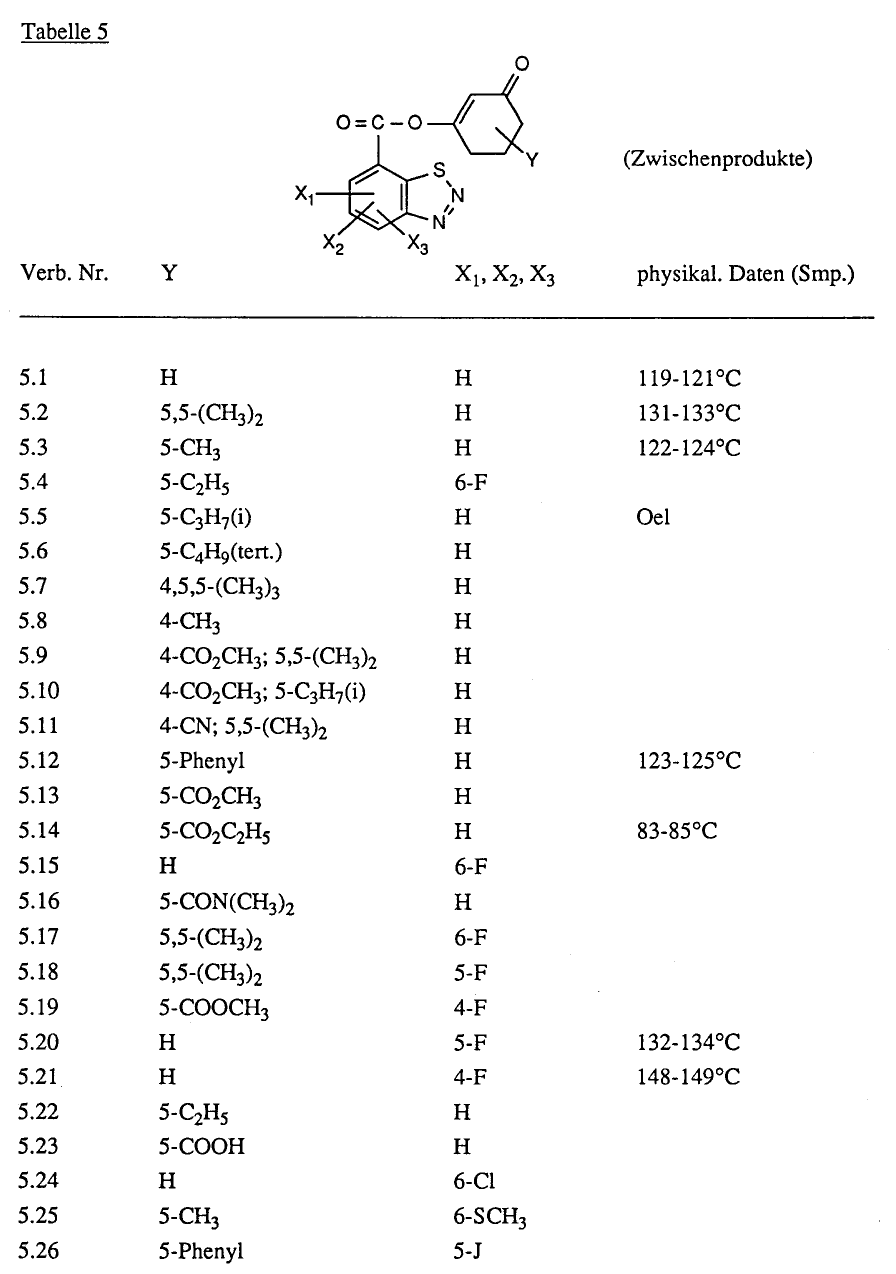

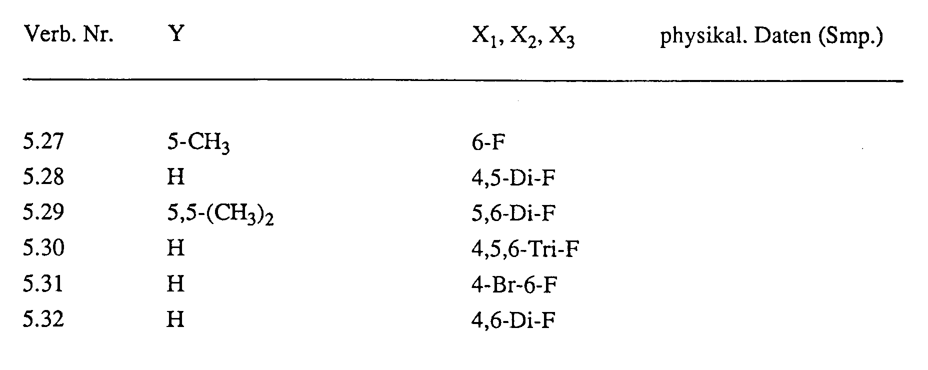

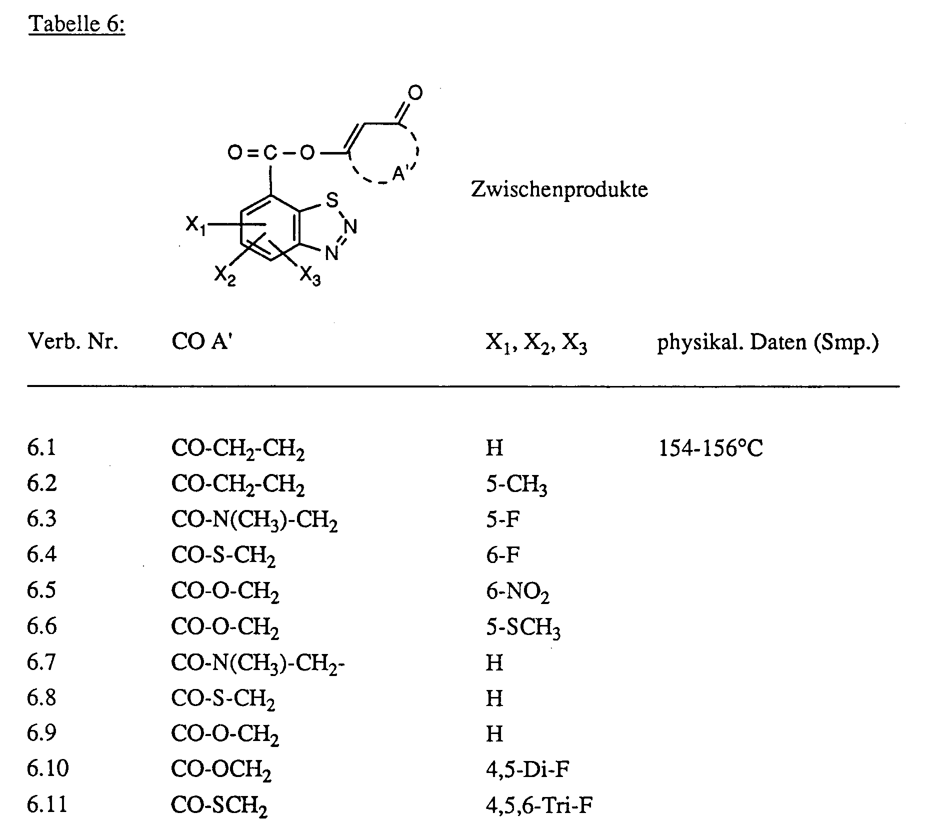

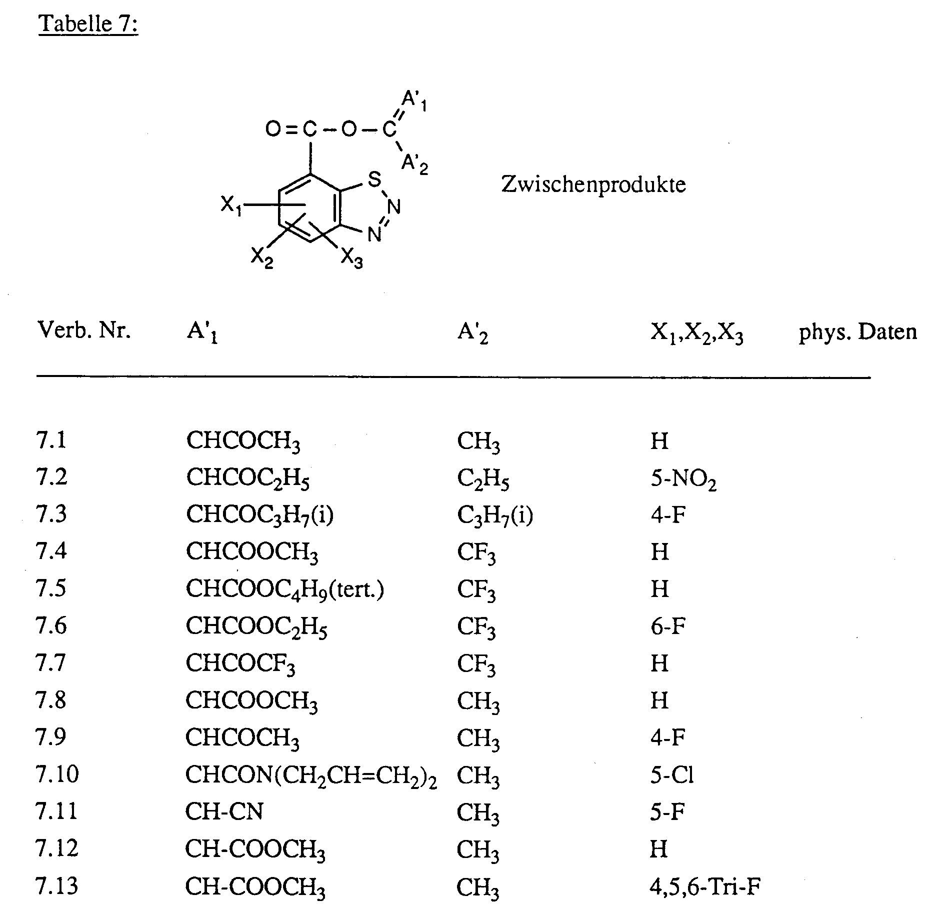

### Formulierungsbeispiele für Wirkstoffe aus den Tabellen (% = Gewichtsprozent)

### 2.1 Spritzpulver

| | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

### 2.2 Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

### 2.3 Stäubemittel

| | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

### 2.4 Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

### 2.5 Umhüllungs-Granulat

| | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

### 2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

### 3. Biologische Beispiele

### Beispiel 3.1: Wirkung geben Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm). Nach 48 Stunden werden die Pflanzen mit einer Sporensuspension (1.5 · 10⁵ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C bis 23°C weitergeführt.
   Die Beurteilung der Schutzwirkung erfolgt aufgrund des Pilzbefalls 7-8 Tage nach der Infektion.
b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen). Nach 48 Stunden werden die Pflanzen mit einer Sporensuspension (1.5 · 10⁵ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C weitergeführt.

Die Beurteilung der Schutzwirkung erfolgt aufgrund des Pilzbefalls 7-8 Tage nach der Infektion.

Verbindungen aus den Tabellen 1 bis 7 zeigen in den Tests (a) und (b) gute Wirkung. So reduzierten z.B. die Verbindungen 1.1, 1.2, 1.3, 1.5, 1.12, 1.14, 2.2, 2.5, 2.6, 2.12, 3.2, 3.13, 4.2, 4.4, 4.5, 4.14, 4.15, 4.16, 5.1, 5.2, 5.3, 5.12, 5.14, 5.21 und 6.1 den Pilzbefall auf 0 bis 20 %. Unbehandelte jedoch infizierte Kontrollpflanzen weisen dagegen einen Colletotrichum-Befall von 100 % auf.

### Beispiel 3.2: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 9O-100 % relativer Luftfeuchtigkeit und 20°C.
b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen 1 bis 7 zeigen gegen den Phytophthora-Pilz eine gute Schutzwirkung. So reduzierten z.B. im Test (a) die Verbindungen 2.5, 4.15, 5.1, 5.14 und 6.1 und im Test (b) die Verbindungen 2.5, 3.2, 5.1, 5.12 und 6.1 den Pilzbefall auf 0 bis 20 %. Unbehandelte jedoch infizierte Kontrollpflanzen weisen dagegen einen 100 %igen Phytophthora-Befall auf.

### Beispiel 3.3: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt.
b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.

Reispflanzen, die mit einer Spritzbrühe behandelt werden, die als Aktivsubstanz eine Verbindung aus den Tabellen 1 bis 7 enthält, zeigen im Vergleich zu unbehandelten Kontrollpflanzen (100 % Befall) nur geringen Pilzbefall. So reduzierten z.B. im Test (a) die Verbindung 1.1 den Befall auf 0 bis 20 %.

### Beispiel 3.4: Wirkung gegen Bremia letucae auf Salat

Zu zweiwöchigen Salatpflanzen wird eine formulierte Lösung des Wirkstoffes (0,002 % Aktivsubstanz bezogen auf das Erdvolumen) gegossen. Nach 5 Tagen werden die behandelten Pflanzen mit einer Sporensuspension des Pilzes (5 x 10⁴ s/ml) inokuliert. Die Pflanzen werden bei 18°C zuerst unter einer Haube (relative Luftfeuchtigkeit von 90-100 %) während 2 Tagen, dann ohne Haube während 7 Tagen inkubiert. Um den Pilz zur Sporulation zu bringen, werden die Pflanzen wieder für 3 Tage unter eine Haube gestellt.

Die Beurteilung des Pilzbefalls erfolgt 12 Tage nach der Inokulation aufgrund der mit Pilz befallenen Blattoberfläche.

Verbindungen aus den Tabellen 1 bis 7 zeigen eine gute Wirkung gegen Bremia. So blieben Pflanzen, die z.B. mit der Verbindung 1.1 oder 1.3 behandelt wurden, weitgehend befallsfrei (0-30 % Schädigung). Unbehandelte, aber infizierte Pflanzen (Kontrolle) wiesen dagegen einen Bremia-Befall von 100 % auf.

### Beispiel 3.5: Wirkung gegen Erysiphe graminis auf Weizen

Protektive Wirkung: 17 Tage alte Weizenpflanzen werden mit einer formulierten Lösung des Wirkstoffes (0,02 % Aktivsubstanz) besprüht. Unmittelbar nach der Behandlung werden die Pflanzen unter Zylindern inkubiert. Nach 24 Stunden werden die Pflanzen abgedeckt. Nach weiteren 3 Tagen werden die behandelten Pflanzen oberhalb vom Primärblatt abgeschnitten. Die Primärblätter werden horizontal orientiert und in einer Bestäubungsglocke mit Erysiphe graminis-Sporen inokuliert (Sporendichte: 0,2 mg pro m²). Der Test wird in einer Klimakammer bei 12h Licht (18 kLux), 20°C und 12h Dunkelheit, 18°C durchgeführt.

Die Beurteilung des Befalls erfolgt 9 und 13 Tage nach Inokulation.

Als Wirkstoff eingesetzte Verbindungen aus den Tabellen 1 bis 7 zeigen in diesem Test eine gute Wirkung gegen Erysiphe graminis. So blieben Pflanzen, die z.B. mit der Verbindung 1.1, 1.2, 1.3, 1.5, 1.12, 1.14, 1.15, 2.5, 2.12, 3.2, 4.2, 4.4, 4.5, 4.15, 4.16, 5.2, 5.3, 5.14 oder 5.21 behandelt wurden, weitgehend frei von Erysiphe-Befall (0 bis 20 % Schädigung). Unbehandelte aber infizierte Pflanzen (Kontrolle) weisen dagegen einen Erysiphe-Befall von 100 % auf.

### Beispiel 3.6: Wirkung gegen Cercospora nicotianae an Tabakpflanzen

### A) Blattapplikation

Tabakpflanzen (8 Wochen alt) werden mit einer formulierten Lösung des Wirkstoffs besprüht (Konzentration: 200 ppm). Vier Tagen nach der Behandlung werden die Pflanzen mit einer Sporensuspension von Cercospora nicotianae (10⁵ Sporen/ml) inokuliert und für 5 Tage bei hoher Luftfeuchtigkeit und einer Temperatur von 22°-25°C inkubiert. Die Inkubation wird dann bei normaler Luftfeuchtigkeit und bei 20°-22°C weitergeführt.

### B) Bodenapplikation

Tabakpflanzen (8 Wochen alt) wurden mit einer formulierten Lösung des Wirkstoffs durch Bodenapplikation behandelt (Konzentration: 0,002 % Aktivsubstanz). Nach 4 Tagen wurden die Pflanzen mit einer Sporensuspension von Cercospora nicotianae (10⁵ Sporen/ml) inokuliert und für 5 Tage bei hoher Luftfeuchtigkeit und einer Temperatur von 22°-25°C inkubiert. Die Inkubation wurde dann bei normaler Luftfeuchtigkeit und bei 20°-22°C weitergeführt.

Die Beurteilung der Symptome in den Tests A und B erfolgt aufgrund des Pilzbefalls 12 bis 14 Tage nach der Infektion.

Die Kontrollpflanzen zeigten einen Befall von 100 %. Pflanzen, welche z.B. im Test A mit der Verbindung 1.1, 1.2, 1.3, 1.12, 1.14, 1.15, 2.5, 4.2, 4.4, 4.5, 4.15, 4.16, 5.2, 5.3, 5.12 oder 5.14 behandelt wurden, zeigten einen Befall von 0-20 %.

### Beispiel 3.7: Wirkung gegen Cercospora arachidicola auf Erdnusspflanzen

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen 1 bis 7 behandelt wurden, einen stark reduzierten Cercospora-Befall. So verhinderten die Verbindungen Nr. 3.2 und 5.3 in obigen Versuchen das Auftreten von Flecken weitgehend (0-20 %).

### Beispiel 3.8: Wirkung gegen Puccinia graminis auf Weizen Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Unbehandelte aber infizierte Kontrollpflanzen zeigen einen Puccinia-Befall von 100 %. Verbindungen aus den Tabellen 1 bis 7 zeigen gegen Puccinia-Pilze eine gute Wirkung.

So reduzieren z.B. die Verbindungen Nr. 1.1, 1.14, 1.15, 2.5, 2.6, 3.2, 4.4, 4.5, 4.16, 5.1 und 6.1 den Pilzbefall auf unter 20 %.

### Beispiel 3.9: Wirkung gegen Pseudomonas lachrymans an Cucumis sativus L.

### A) Blattapplikation

Gurkenpflanzen werden nach 2-wöchiger Anzucht mit einer aus Spritzpulver des Wirkstoffes hergestellten Spritzbrühe besprüht (Konzentration: 0,02 % Aktivsubstanz).

Nach 1 Woche werden die Pflanzen mit einer Bakteriensuspension (10⁸ Bakterien/ml) infiziert und für 7 Tage bei hoher Luftfeuchtigkeit und einer Temperatur von 23°C inkubiert.

Die Beurteilung der Schutzwirkung erfolgt aufgrund des Bakterienbefalls 7-8 Tage nach der Infektion.

Verbindungen aus den Tabellen 1 bis 7 bewirken einen guten Schutz gegen Pseudomonas lachrymans. So blieben Pflanzen, die z.B. mit der Verbindung 1.1, 1.3, 1.12, 1.14, 1.15, 2.5, 3.2, 4.5, 4.15, 4.16, 5.3 oder 5.14 behandelt wurden, weitgehend frei von Pseudomonas (Befall 20 bis 0 %).

### B) Bodenapplikation

Gurkenpflanzen werden nach 2-wöchiger Anzucht mit einer aus Spritzpulver des Wirkstoffes hergestellten Spritzbrühe durch Bodenapplikation behandelt (Konzentration: 0,002 % Aktivsubstanz bezogen auf das Bodenvolumen).

Nach 1 Woche werden die Pflanzen mit einer Bakteriensuspension (10⁸ Bakterien/ml) infiziert und für 7 Tage bei hoher Luftfeuchtigkeit und einer Temperatur von 23°C inkubiert.

Die Beurteilung der Schutzwirkung erfolgt aufgrund des Befalls 7-8 Tage nach der Infektion.

Verbindungen aus den Tabellen 1 bis 7 bewirken eine gute Immunisierung gegen Pseudomonas lachrymans. So blieben Pflanzen, die z.B. mit der Verbindung 1.1, 1.2, 1.3, 1.12, 1.14, 1.15, 2.5, 3.2, 4.2, 4.4, 4.5, 4.15, 4.16, 5.2, 5.3, 5.12 oder 5.14 behandelt wurden, fast völlig frei von Pseudomonas (Befall 20 bis 0 %).

Unbehandelte aber infizierte Kontrollpflanzen weisen in den Tests A und B einen Krankheitsbefall von 100 % auf.

### Beispiel 3.10: Wirkung gegen Plasmopara viticola an Reben

Im 4-5 Blattstadium werden Rebensämlinge mit einer aus Spritzpulver des Wirkstoffes hergestellten Spritzbrühe (0,02 % Aktivsubstanz) besprüht. Nach 1 Woche werden die behandelten Pflanzen mit einer Sporangiensuspension 5·10⁴ Sporangien/ml) des Pilzes infiziert. Nach einer Inkubation während 6 Tagen bei 95-100 % relativer Luftfeuchtigkeit und 20°C wird die Schutzwirkung beurteilt.

Unbehandelte aber infizierte Kontrollpflanzen weisen in diesem Test einen Befall von 100 % auf.

Verbindungen aus den Tabellen 1 bis 7 bewirken eine gute Wirkung gegen Plasmopara viticola. So blieben Reben, die z.B. mit der Verbindung 2.5 oder 5.1 behandelt wurden, weitgehend frei von Plasmopara viticola (Befall 20 bis 0 %).

### Beispiel 3.11: Wirkung gegen Pythium ultimum auf Zea mays (Mais, cv. Sweet Corn)

Testprinzip: Bodenpilz: protektiv lokale Bodenapplikation

Testmethode: Myzel von Pythium ultimum wird mit Erde gemischt (500 ml Myzelsuspension auf 10 Liter Erde) und das Pilz-Erdgemisch in 250 ml Plastikschalen abgefüllt. Nach einer 4-tägigen Inkubation bei 10°C werden pro Schale 10 Körner der zu prüfenden Pflanze (Mais) gesteckt. Am nächsten Tag werden die so vorbereiteten Schalen mit je 50 ml, aus 25 % Spritzpulver und Wasser hergestellten Spritzlösungen mit 20; 6; 2; 0,6; 0,2; 0,06 und 0,02 ppm AS übergossen. Nach einer 7-tägigen Inkubationsphase bei 10°C und einer anschliessenden 4-tägigen Inkubationsphase bei 22°C wird die Wirkung der Testsubstanzen durch zahlenmässige Bestimmung des Auflaufs der Testpflanzen bewertet.
Verbindungen aus den Tabellen 1 bis 7 erzielen gegen Pythium ultimum gute Wirkung. So zeigten die Verbindungen 2.5 oder 4.5 eine Wirkung von mehr als 80 %.

### Beispiel 3.12: Wirkung gegen Peronospora tabacina an Tabakpflanzen

### A) Blattapplikation

Tabakpflanzen (8 Wochen alt) werden mit einer formulierten Lösung des Wirkungsstoffes besprüht (Konzentration: 0,02 % Aktivsubstanz). Vier Tage nach der Behandlung werden die Pflanzen mit einer Sporangiensuspension von Peronospora tabacina (10⁴ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

### B) Bodenapplikation

Tabakpflanzen (8 Wochen alt) werden mit einer formulierten Lösung des Wirkungsstoffes durch Bodenapplikation behandelt (Konzentration: 0,006 % Aktivsubstanz bezogen auf das Bodenvolumen). Nach 4 Tagen werden die Pflanzen mit einer Sporangiensuspension von Peronospora tabacina (10⁴ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

Die Beurteilung der Symptome in den Tests A und B erfolgt aufgrund der mit Pilz befallenen Blattoberfläche.

Die Kontrollpflanzen zeigen einen Befall von 90 bis 100 %. Pflanzen, welche in Test A mit der Verbindung 1.1 behandelt wurden, zeigten einen Befall von 0-30 %.

## Patentansprüche (Patentansprüche für folgende(n) Vertragsstaat(en): CH, DE, DK, FR, GB, IT, LI, NL)

1. Verbindungen der Formel I 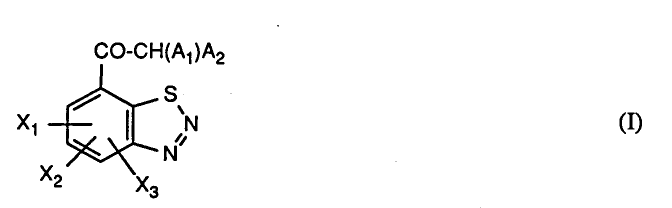 in welcher bedeuten:
A₁ und A₂ unabhängig voneinander CO-C₁-C₄-Alkyl, COO-C₁-C₄-Alkyl, CO-CF₃, CO-N(R)₂ oder Cyano;
A₁ und A₂ zusammen CO(X)ₙ-C₁-C₃-Alkylen-(X)ₙCO, durch C₁-C₄-Alkyl, COOR, CON(R)₂, Cyano oder Phenyl substituiertes CO(X)ₙ-C₁-C₃-Alkylen-(X)ₙCO, wobei der Phenylring seinerseits durch Halogen, Methyl, Trifluormethyl, Methoxy, Nitro oder Cyano substituiert sein kann; CO-N(R)-CO-N(R)-CO;
R Wasserstoff, C₁-C₆-Alkyl, C₃-C₆-Alkenyl oder C₃-C₆-Alkinyl;
X Sauerstoff, Schwefel oder N(CH₃);
X₁, X₂, X₃ unabhnängig voneinander Wasserstoff, Halogen, Methyl, Methylthio, Methoxy oder Nitro;
n 0 oder 1;
unter Einschluss der Salze der Verbindungen der Formel I mit pflanzenverträglichen organischen oder anorganischen Basen sowie der Metallkomplexe.

2. Verbindungen gemäss Anspruch 1 der Formel I, in welcher bedeuten:
A₁ und A₂ zusammen CO(X)ₙ-C₁-C₃-Alkylen-(X)ₙCO, durch C₁-C₄-Alkyl, COOH, COOCH₃, COOC₂H₅, COOCH₂-CH=CH₂, COOCH₂-C≡CH, Phenyl, die Gruppe CO-N(CH₃)₂ oder Cyano substituiertes CO-C₁-C₃-Alkylen-CO; CO-N(R')-CO-N(R')-CO;
R' Wasserstoff, C₁-C₃-Alkyl;
X Sauerstoff oder Schwefel;
X₁, X₂, X₃ unabhängig voneinander Wasserstoff, F, Methyl, Methoxy, Methylthio oder Nitro;
n 0 oder 1;
unter Einschluss der Salze der Verbindungen der Formel I mit organischen oder anorganischen Basen sowie der Metallkomplexe mit Mg, Ca, Ba, Sn, Fe, Zn, Cu, Ni oder Mn.

3. Verbindungen gemäss Anspruch 1 der Formel I, in welcher bedeuten:
A₁ und A₂ unabhängig voneinander CO-C₁-C₄-Alkyl, COO-C₁-C₄-Alkyl, CO-CF₃, CO-N(R)₂ oder Cyano;
R Wasserstoff, C₁-C₆-Alkyl, C₃-C₆-Alkenyl oder C₃-C₆-Alkinyl;
X₁, X₂, X₃ unabhängig voneinander Wasserstoff, Halogen, Methyl, Methylthio, Methoxy oder Nitro;
unter Einschluss der Salze der Verbindungen der Formel I mit pflanzenverträglichen organischen oder anorganischen Basen sowie der Metallkomplexe mit Mg, Ca, Ba, Cu, Sn, Fe, Zn, Ni oder Mn.

4. Verbindungen der Formel I gemäss Anspruch 2, in welcher bedeuten:
A₁ und A₂ zusammen CO(X)ₙ-C₁-C₃-Alkylen-(X)ₙCO, durch C₁-C₄-Alkyl, COOH,COOCH₃, Phenyl oder die Gruppe CO-N(CH₃)₂ substituiertes CO(X)ₙ-C₁-C₃-Alkylen-(X)ₙCO;
X Sauerstoff;
X₁, X₂, X₃ unabhängig voneinander Wasserstoff, Fluor oder Methyl;
n 0 oder 1.

5. Verbindungen der Formel I gemäss Anspruch 3, in welcher bedeuten:
A₁ und A₂ unabhängig voneinander CO-C₁-C₂-Alkyl, COO-C₁-C₂-Alkyl, CO-CF₃ oder CO-N(C₁-C₂-Alkyl)₂;
X₁, X₂, X₃ unabhängig voneinander Wasserstoff, Fluor oder Methyl.

6. Verbindungen der Formel I gemäss Anspruch 4, in welcher bedeuten:
A₁ und A₂ zusammen CO-C₁-C₃-Alkylen-CO, durch C₁-C₂-Alkyl, COOH oder Phenyl substituiertes CO-C₁-C₃-Alkylen-CO;
X₁, X₂, X₃ unabhängig voneinander Wasserstoff oder Fluor.

7. Verbindungen der Formel I gemäss Anspruch 5, in welcher bedeuten:
A₁ und A₂ unabhängig voneinander CO-C₁-C₂-Alkyl, COO-C₁-C₂-Alkyl oder CO-N(CH₃)₂;
X₁, X₂, X₃ unabhängig voneinander Wasserstoff oder Fluor.

8. Verbindungen der Formel I gemäss Anspruch 6, in welcher bedeuten:
A₁ und A₂ zusammen CO-C₁-C₃-Alkylen-CO, durch Methyl substituiertes CO-C₁-C₃-Alkylen-CO;
X₁, X₂, X₃ unabhängig voneinander Wasserstoff oder Fluor.

9. Verbindungen der Formel I gemäss Anspruch 3, in welcher bedeuten:
A₁ und A₂ unabhängig voneinander CO-CH₃, COOC₁-C₄-Alkyl, CN oder CON(CH₂C≡CH)₂;
X₁, X₂, X₃ unabhängig voneinander Wasserstoff oder Fluor.

10. Verbindungen der Formel I gemäss Anspruch 2, in welcher bedeuten:
A₁ und A₂ zusammen CO-N(R′)-CO-N(R′)-CO;
R′ Wasserstoff, C₁-C₃-Alkyl
X₁, X₂, X₃ unabhängig voneinander Wasserstoff, Fluor oder Methyl.

11. Verbindungen der Formel I gemäs Anspruch 10, in welchem bedeuten:
A₁ und A₂ zusammen CO-N(CH₃)-CO-N(CH₃)-CO;
X₁, X₂, X₃ unabhängig voneinander Wasserstoff oder Fluor.

12. Eine Verbindung der Formel I gemäss Anspruch 1 ausgewählt aus der Gruppe bestehend aus
2-(Benzo-1,2,3-thiadiazol-7-carbonyl)-3-hydroxy-cyclohex-2-enon;
2-(Benzo-1,2,3-thiadiazol-7-carbonyl)-3-hydroxy-5,5-dimethyl-cyclohex-2-enon;
2-(Benzo-1,2,3-thiadiazol-7-carbonyl)-3-hydroxy-5-methyl-cyclohex-2-enon;
2-(Benzo-1,2,3-thiadiazol-7-carbonyl)-3-hydroxy-5-isopropyl-cyclohex-2-enon;
2-(Benzo-1,2,3-thiadiazol-7-carbonyl)-3-hydroxy-5-phenyl-cyclohex-2-enon;
2-(6-Fluor-benzo-1,2,3-thiadiazol-7-carbonyl)-3-hydroxy-5,5-dimethyl-cyclohex-2-enon;
2-(4-Fluor-benzo-1,2,3-thiadiazol-7-carbonyl)-3-hydroxy-cyclohex-2-enon;
5-(Benzo-1,2,3-thiadiazol-7-carbonyl)-1,3-dimethyl-2,4,6-1H,3H,5H-pyrimidintrion und (Benzo-1,2,3-thiadiazol-7-carbonyl)-malonsäurediethylester.

13. Verfahren zur Herstellung von Verbindungen gemäss Anspruch 1 der Formel I gekennzeichnet:
a) durch Acylierung von Methylenverbindungen der Formel II
A₁-CH₂-A₂ (II)
mit einem aktivierten Säurederivat der Formel III 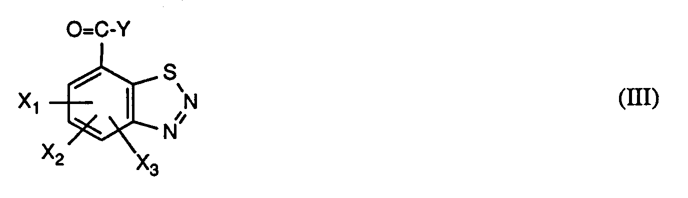 mit oder ohne Zusatz von Metallsalzen, wie MgCl₂ oder MgO, in Gegenwart einer Base oder einer Lewis-Säure in inerten Lösungsmitteln bei Temperaturen von -30°C bis 180°C, wobei Y Halogen, Cyano oder den Rest 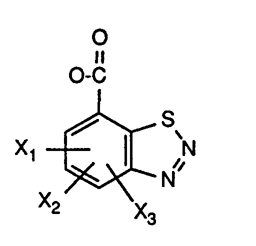 darstellt und A₁, A₂, X₁, X₂ und X₃ die unter Formel I angegebenen Bedeutungen besitzen; oder
b) aus Methylenverbindungen der Formel IIa 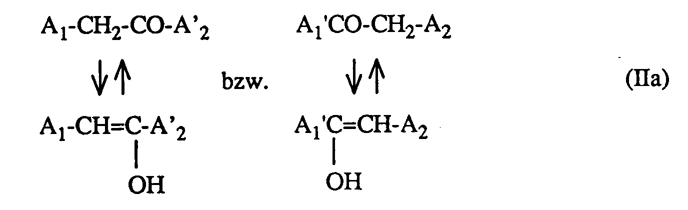 durch Acylierung der Enolform am Sauerstoffatom mit einer Verbindung der Formel III 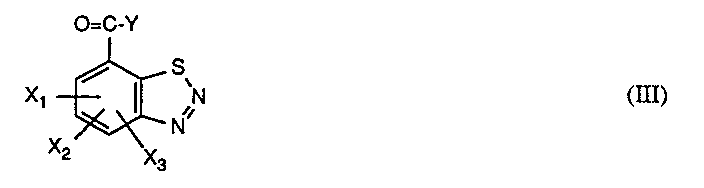 in Gegenwart einer Base in einem inerten Lösungsmittel bei Temperaturen von - 30°C bis 160°C über die Zwischenprodukte der Formel IV 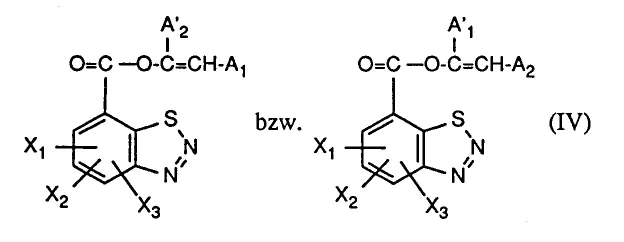 wobei A₁′ bzw. A₂′ folgende Bedeutungen hat:
C₁-C₄-Alkyl, O-C₁-C₄-Alkyl, CF₃, N(R)₂, unsubstituiertes oder durch C₁-C₄-Alkyl, COOR, CON(R)₂, Cyano oder Phenyl substituiertes (X)ₙ-C₁-C₃-Alkylen-(X)ₙCO, wobei der Phenylring seinerseits durch Halogen, Methyl, Trifluormethyl, Methoxy, Nitro oder Cyano substituiert sein kann; und N(R)-CO-N(R)-CO, wobei R Wasserstoff, C₁-C₆-Alkyl, C₃-C₆-Alkenyl oder C₃-C₆-Alkinyl bedeutet;
und diese mit einer Base mit oder ohne Zusatz von Cyanid-Verbindungen oder einer Lewis-Säure, wie z.B. AlCl₃, als Katalysator in inerten Lösungsmitteln bei Temperaturen von -30°C bis 160°C, insbesondere 0°C bis 120°C in Verbindungen der Formel Ia 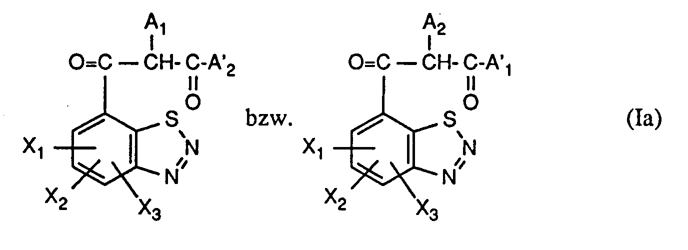 umlagert, wobei die Verbindungen der Formel I und Ia auch in den Enolformen der Formel Ia′ 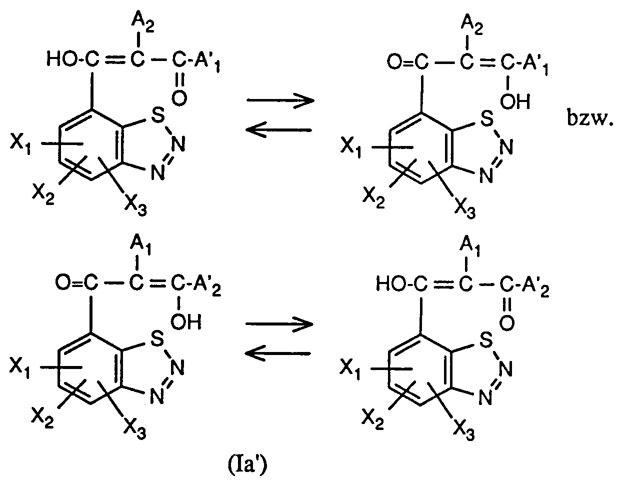 vorliegen können; und wobei X₁, X₂, X₃, A₁, A₂, A₁′ und A₂′ die unter Formel I, II und IV angegebenen Bedeutungen besitzen.

14. Mittel zur Bekämpfung oder Verhütung eines Befalls durch schädliche Mikroorganismen, dadurch gekennzeichnet, dass es als aktive Komponente mindestens eine Verbindung der Formel I gemäss Anspruch 1 enthält, zusammen mit einem geeigneten Trägermaterial.

15. Mittel gemäss Anspruch 14, dadurch gekennzeichnet, dass es als aktive Komponente mindestens eine Verbindung der Formel I gemäss Anspruch 2 enthält.

16. Mittel gemäss Anspruch 14, dadurch gekennzeichnet, dass es als aktive Komponente mindestens eine Verbindung der Formel I gemäss Anspruch 3 enthält.

17. Mittel gemäss Anspruch 14, dadurch gekennzeichnet, dass es als aktive Komponente mindestens eine Verbindung der Formel I gemäss einem der Ansprüche 4 bis 11 enthält.

18. Mittel gemäss Anspruch 14, dadurch gekennzeichnet, dass es als aktive Komponente mindestens eine Verbindung der Formel I gemäss Anspruch 12 enthält.

19. Verfahren zur Bekämpfung oder Verhütung eines Befalls von Kulturpflanzen durch phytopathogene Mikroorganismen, dadurch gekennzeichnet, dass man als Wirkstoff eine Verbindung der Formel I gemäss Anspruch 1 auf die Pflanze, auf Pflanzenteile oder ihren Standort appliziert.

20. Verfahren gemäss Anspruch 19, dadurch gekennzeichnet, dass man als Wirkstoff eine Verbindung gemäss einem der Ansprüche 2 bis 11 appliziert.

21. Verfahren gemäss Anspruch 19, dadurch gekennzeichnet, dass man als Wirkstoff eine Verbindung gemäss Anspruch 12 appliziert.

22. Verfahren gemäss Anspruch 19, dadurch gekennzeichnet, dass phytopathogene Fungi bekämpft werden.

23. Verwendung von Verbindungen der Formel I gemäss Anspruch 1 zur Bekämpfung oder Verhütung eines Befalls von Kulturpflanzen durch phytopathogene Mikroorganismen, dadurch gekennzeichnet, dass man als Wirkstoff eine Verbindung der Formel I auf die Pflanze, auf Pflanzenteile oder ihren Standort appliziert.

24. Verfahren zur Herstellung eines agrochemischen Mittels gemäss Anspruch 14, dadurch gekennzeichnet, dass man die Komponenten innig vermischt.

## Patentansprüche (Patentansprüche für folgende(n) Vertragsstaat(en): ES)

1. Verfahren zur Herstellung der Verbindungen der Formel I 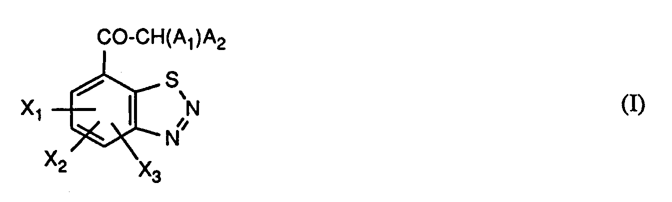 in welcher bedeuten:
A₁ und A₂ unabhängig voneinander CO-C₁-C₄-Alkyl, COO-C₁-C₄-Alkyl, CO-CF₃, CO-N(R)₂ oder Cyano;
A₁ und A₂ zusammen CO(X)ₙ-C₁-C₃-Alkylen-(X)ₙCO, durch C₁-C₄-Alkyl, COOR, CON(R)₂, Cyano oder Phenyl substituiertes CO(X)ₙ-C₁-C₃-Alkylen-(X)ₙCO, wobei der Phenylring seinerseits durch Halogen, Methyl, Trifluormethyl, Methoxy, Nitro oder Cyano substituiert sein kann; CO-N(R)-CO-N(R)-CO;
R Wasserstoff, C₁-C₆-Alkyl, C₃-C₆-Alkenyl oder C₃-C₆-Alkinyl;
X Sauerstoff, Schwefel oder N(CH₃);
X₁, X₂, X₃ unabhnängig voneinander Wasserstoff, Halogen, Methyl, Methylthio, Methoxy oder Nitro;
n 0 oder 1;
unter Einschluss der Salze der Verbindungen der Formel I mit pflanzenverträglichen organischen oder anorganischen Basen sowie der Metallkomplexe, dadurch gekennzeichnet, dass man
a) eine Methylenverbindung der Formel II
A₁-CH₂-A₂ (II)
mit einem aktivierten Säurederivat der Formel III 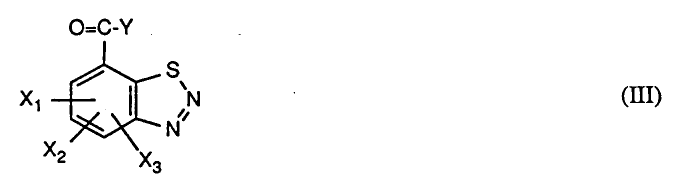 mit oder ohne Zusatz von Metallsalzen, wie MgCl₂ oder MgO, in Gegenwart einer Base oder einer Lewis-Säure in inerten Lösungsmitteln bei Temperaturen von -30°C bis 180°C, wobei Y Halogen, Cyano oder den Rest 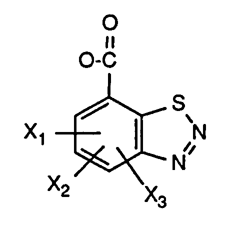 darstellt und A₁, A₂, X₁, X₂ und X₃ die unter Formel I angegebenen Bedeutungen besitzen, acyliert; oder
b) durch Acylierung der Enolform von Methylenverbindungen der Formel IIa 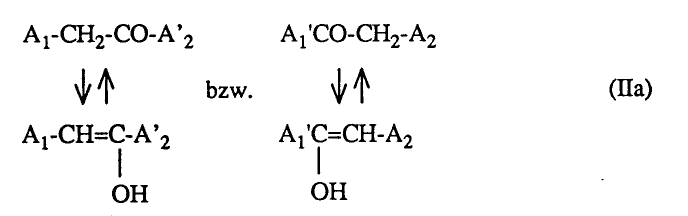 am Sauerstoffatom mit einer Verbindung der Formel III 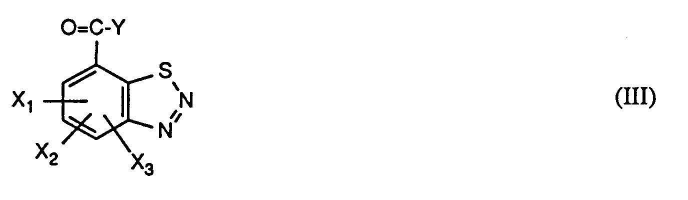 in Gegenwart einer Base in einem inerten Lösungsmittel bei Temperaturen von -30°C bis 160°C Zwischenprodukte der Formel IV 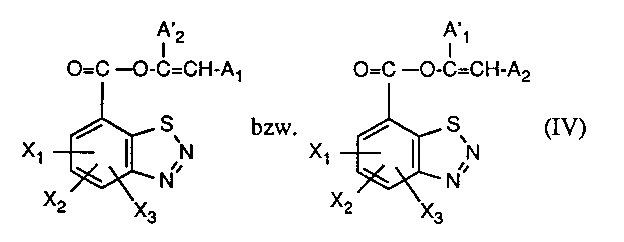 erhält, wobei A₁′ bzw. A₂′ folgende Bedeutungen hat:
C₁-C₄-Alkyl, O-C₁-C₄-Alkyl, CF₃, N(R)₂, unsubstituiertes oder durch C₁-C₄-Alkyl, COOR, CON(R)₂, Cyano oder Phenyl substituiertes (X)ₙ-C₁-C₃-Alkylen-(X)ₙCO, wobei der Phenylring seinerseits durch Halogen, Methyl, Trifluormethyl, Methoxy, Nitro oder Cyano substituiert sein kann; und N(R)-CO-N(R)-CO, wobei R Wasserstoff, C₁-C₆-Alkyl, C₃-C₆-Alkenyl oder C₃-C₆-Alkinyl bedeutet;
und diese mit einer Base mit oder ohne Zusatz von Cyanid-Verbindungen oder einer Lewis-Säure, wie z.B. AlCl₃, als Katalysator in inerten Lösungsmitteln bei Temperaturen von -30°C bis 160°C, insbesondere 0°C bis 120°C in Verbindungen der Formel Ia 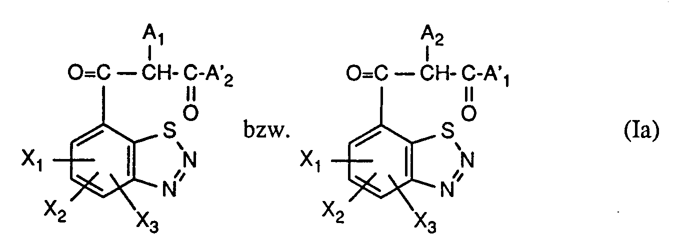 umlagert, wobei die Verbindungen der Formel I und Ia auch in den Enolformen der Formel Ia′ 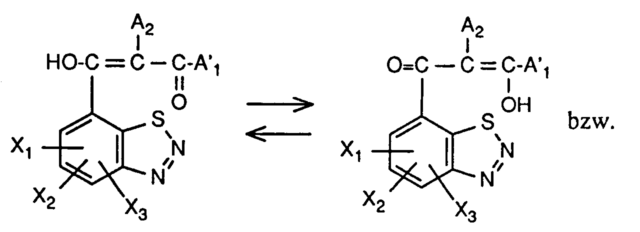 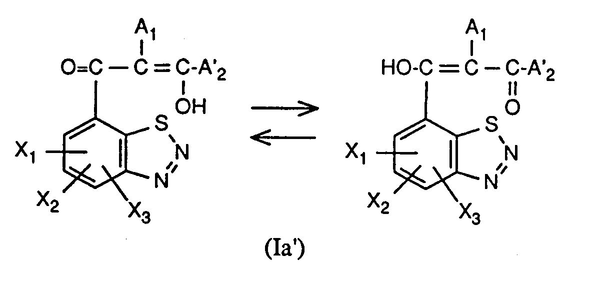 vorliegen können; und wobei X₁, X₂, X₃, A₁, A₂, A₁′ und A₂′ die unter Formel I, II und IV angegebenen Bedeutungen besitzen.

2. Verfahren gemäss Anspruch 1 zur Herstellung von Verbindungen der Formel I worin bedeuten:
A₁ und A₂ zusammen CO(X)ₙ-C₁-C₃-Alkylen-(X)ₙCO, durch C₁-C₄-Alkyl, COOH, COOCH₃, COOC₂H₅, COOCH₂-CH=CH₂, COOCH₂-C≡CH, Phenyl, die Gruppe CO-N(CH₃)₂ oder Cyano substituiertes CO-C₁-C₃-Alkylen-CO; CO-N(R′)-CO-N(R′)-CO;
R′ Wasserstoff, C₁-C₃-Alkyl;
X Sauerstoff oder Schwefel;
X₁, X₂, X₃ unabhängig voneinander Wasserstoff, F, Methyl, Methoxy, Methylthio oder Nitro;
n 0 oder 1;
unter Einschluss der Salze der Verbindungen der Formel I mit organischen oder anorganischen Basen sowie der Metallkomplexe mit Mg, Ca, Ba, Sn, Fe, Zn, Cu, Ni oder Mn.

3. Verfahren gemäss Anspruch 1 zur Herstellung von Verbindungen der Formel I, worin bedeuten:
A₁ und A₂ unabhängig voneinander CO-C₁-C₄-Alkyl, COO-C₁-C₄-Alkyl, CO-CF₃, CO-N(R)₂ oder Cyano;
R Wasserstoff, C₁-C₆-Alkyl, C₃-C₆-Alkenyl oder C₃-C₆-Alkinyl;
X₁, X₂, X₃ unabhängig voneinander Wasserstoff, Halogen, Methyl, Methylthio, Methoxy oder Nitro;
unter Einschluss der Salze der Verbindungen der Formel I mit pflanzenverträglichen organischen oder anorganischen Basen sowie der Metallkomplexe mit Mg, Ca, Ba, Cu, Sn, Fe, Zn, Ni oder Mn.

4. Verfahren gemäss Anspruch 2 zur Herstellung von Verbindungen der Formel I, worin bedeuten:
A₁ und A₂ zusammen CO(X)ₙ-C₁-C₃-Alkylen-(X)ₙCO, durch C₁-C₄-Alkyl, COOH, COOCH₃, Phenyl oder die Gruppe CO-N(CH₃)₂ substituiertes CO(X)ₙ-C₁-C₃-Alkylen-(X)ₙCO;
X Sauerstoff;
X₁, X₂, X₃ unabhängig voneinander Wasserstoff, Fluor oder Methyl;
n 0 oder 1.

5. Verfahren gemäss Anspruch 3 zur Herstellung von Verbindungen der Formel I worin bedeuten:
A₁ und A₂ unabhängig voneinander CO-C₁-C₂-Alkyl, COO-C₁-C₂-Alkyl, CO-CF₃ oder CO-N(C₁-C₂-Alkyl)₂;
X₁, X₂, X₃ unabhängig voneinander Wasserstoff, Fluor oder Methyl.

6. Verfahren gemäss Anspruch 4 zur Herstellung von Verbindungen der Formel I worin bedeuten:
A₁ und A₂ zusammen CO-C₁-C₃-Alkylen-CO, durch C₁-C₂-Alkyl, COOH oder Phenyl substituiertes CO-C₁-C₃-Alkylen-CO;
X₁, X₂, X₃ unabhängig voneinander Wasserstoff oder Fluor.

7. Verfahren gemäss Anspruch 5 zur Herstellung von Verbindungen der Formel I worin bedeuten:
A₁ und A₂ unabhängig voneinander CO-C₁-C₂-Alkyl, COO-C₁-C₂-Alkyl oder CO-N(CH₃)₂;
X₁, X₂, X₃ unabhängig voneinander Wasserstoff oder Fluor.

8. Verfahren gemäss Anspruch 6 zur Herstellung von Verbindungen der Formel I worin bedeuten:
A₁ und A₂ zusammen CO-C₁-C₃-Alkylen-CO, durch Methyl substituiertes CO-C₁-C₃-Alkylen-CO;
X₁, X₂, X₃ unabhängig voneinander Wasserstoff oder Fluor.

9. Verfahren gemäss Anspruch 3 zur Herstellung von Verbindungen der Formel I worin bedeuten:
A₁ und A₂ unabhängig voneinander CO-CH₃, COOC₁-C₄-Alkyl, CN oder CON(CH₂C≡CH)₂;
X₁, X₂, X₃ unabhängig voneinander Wasserstoff oder Fluor.

10. Verfahren gemäss Anspruch 2 zur Herstellung von Verbindungen der Formel I worin bedeuten:
A₁ und A₂ zusammen CO-N(R′)-CO-N(R′)-CO;
R′ Wasserstoff, C₁-C₃-Alkyl
X₁, X₂, X₃ unabhängig voneinander Wasserstoff, Fluor oder Methyl.

11. Verfahren gemäss Anspruch 10 zur Herstellung von Verbindungen der Formel I worin bedeuten:
A₁ und A₂ zusammen CO-N(CH₃)-CO-N(CH₃)-CO;
X₁, X₂, X₃ unabhängig voneinander Wasserstoff oder Fluor.

12. Verfahren gemäss Anspruch 1 zur Herstellung einer Verbindung der Formel I, ausgewählt aus der Gruppe bestehend aus
2-(Benzo-1,2,3-thiadiazol-7-carbonyl)-3-hydroxy-cyclohex-2-enon;
2-(Benzo-1,2,3-thiadiazol-7-carbonyl)-3-hydroxy-5,5-dimethyl-cyclohex-2-enon;
2-(Benzo-1,2,3-thiadiazol-7-carbonyl)-3-hydroxy-5-methyl-cyclohex-2-enon;
2-(Benzo-1,2,3-thiadiazol-7-carbonyl)-3-hydroxy-5-isopropyl-cyclohex-2-enon;
2-(Benzo-1,2,3-thiadiazol-7-carbonyl)-3-hydroxy-5-phenyl-cyclohex-2-enon;
2-(6-Fluor-benzo-1,2,3-thiadiazol-7-carbonyl)-3-hydroxy-5,5-dimethyl-cyclohex-2-enon;
2-(4-Fluor-benzo-1,2,3-thiadiazol-7-carbonyl)-3-hydroxy-cyclohex-2-enon;
5-(Benzo-1,2,3-thiadiazol-7-carbonyl)-1,3-dimethyl-2,4,6-1H,3H,5H-pyrimidintrion und (Benzo-1,2,3-thiadiazol-7-carbonyl)-malonsäurediethylester.

13. Mittel zur Bekämpfung oder Verhütung eines Befalls durch schädliche Mikroorganismen, dadurch gekennzeichnet, dass es als aktive Komponente mindestens eine Verbindung der Formel I erhältlich gemäss Anspruch 1 enthält, zusammen mit einem geeigneten Trägermaterial.

14. Mittel gemäss Anspruch 13, dadurch gekennzeichnet, dass es als aktive Komponente mindestens eine Verbindung der Formel I erhältlich gemäss Anspruch 2 enthält.

15. Mittel gemäss Anspruch 13, dadurch gekennzeichnet, dass es als aktive Komponente mindestens eine Verbindung der Formel I erhältlich gemäss Anspruch 3 enthält.

16. Mittel gemäss Anspruch 13, dadurch gekennzeichnet, dass es als aktive Komponente mindestens eine Verbindung der Formel I erhältlich gemäss einem der Ansprüche 4 bis 11 enthält.

17. Mittel gemäss Anspruch 13, dadurch gekennzeichnet, dass es als aktive Komponente mindestens eine Verbindung der Formel I erhältlich gemäss Anspruch 12 enthält.

18. Verfahren zur Bekämpfung oder Verhütung eines Befalls von Kulturpflanzen durch phytopathogene Mikroorganismen, dadurch gekennzeichnet, dass man als Wirkstoff eine Verbindung der Formel I erhältlich gemäss Anspruch 1 auf die Pflanze, auf Pflanzenteile oder ihren Standort appliziert.

19. Verfahren gemäss Anspruch 18, dadurch gekennzeichnet, dass man als Wirkstoff eine Verbindung erhältlich gemäss einem der Ansprüche 2 bis 11 appliziert.

20. Verfahren gemäss Anspruch 18, dadurch gekennzeichnet, dass man als Wirkstoff eine Verbindung erhältlich gemäss Anspruch 12 appliziert.

21. Verfahren gemäss Anspruch 18, dadurch gekennzeichnet, dass phytopathogene Fungi bekämpft werden.

22. Verwendung von Verbindungen der Formel I erhältlich gemäss Anspruch 1 zur Bekämpfung oder Verhütung eines Befalls von Kulturpflanzen durch phytopathogene Mikroorganismen, dadurch gekennzeichnet, dass man als Wirkstoff eine Verbindung der Formel I auf die Pflanze, auf Pflanzenteile oder ihren Standort appliziert.

23. Verfahren zur Herstellung eines agrochemischen Mittels gemäss Anspruch 13, dadurch gekennzeichnet, dass man die Komponenten innig vermischt.

## Claims (Claims for the following Contracting State(s): CH, DE, DK, FR, GB, IT, LI, NL)

1. A compound of the formula I 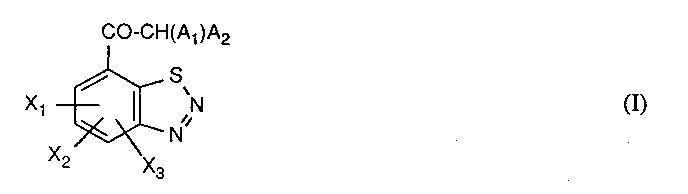 in which:
A₁ and A₂ independently of one another are CO-C₁-C₄alkyl, COO-C₁-C₄alkyl, CO-CF₃, CO-N(R)₂ or cyano;
A₁ and A₂ together are CO(X)ₙ-C₁-C₃alkylene-(X)ₙCO, CO(X)ₙ-C₁-C₃alkylene-(X)ₙCO which is substituted by C₁-C₄alkyl, COOR, CON(R)₂, cyano or phenyl, it being possible for the phenyl ring, in turn, to be substituted by halogen, methyl, trifluoromethyl, methoxy, nitro or cyano; CO-N(R)-CO-N(R)-CO;
R is hydrogen, C₁-C₆alkyl, C₃-C₆alkenyl or C₃-C₆alkynyl;
X is oxygen, sulfur or N(CH₃);
X₁, X₂ and X₃ independently of one another are hydrogen, halogen, methyl, methylthio, methoxy or nitro;
n is 0 or 1;
including the salts of the compound of the formula I with agriculturally acceptable organic or inorganic bases, and including the metal complexes.

2. A compound according to claim 1, of the formula I, in which:
A₁ and A₂ together are CO(X)ₙ-C₁-C₃alkylene-(X)ₙCO, or CO-C₁-C₃alkylene-CO which is substituted by C₁-C₄alkyl, COOH, COOCH₃, COOC₂H₅, COOCH₂-CH=CH₂, COOCH₂-C≡CH, phenyl, the group CO-N(CH₃)₂ or cyano; CO-N(R')-CO-N(R')-CO;
R' is hydrogen or C₁-C₃alkyl;
X is oxygen or sulfur;
X₁, X₂ and X₃ independently of one another are hydrogen, F, methyl, methoxy, methylthio or nitro;
n is 0 or 1;
including the salts of the compound of the formula I with organic or inorganic bases as well as the metal complexes with Mg, Ca, Ba, Sn, Fe, Zn, Cu, Ni or Mn.

3. A compound according to claim 1, of the formula I, in which:
A₁ and A₂ independently of one another are CO-C₁-C₄alkyl, COO-C₁-C₄alkyl, CO-CF₃, CO-N(R)₂ or cyano;
R is hydrogen, C₁-C₆alkyl, C₃-C₆alkenyl or C₃-C₆alkynyl;
X₁, X₂, and X₃ independently of one another are hydrogen, halogen, methyl, methylthio, methoxy or nitro;
including the salts of the compound of the formula I with agriculturally acceptable organic or inorganic bases as well as the metal complexes with Mg, Ca, Ba, Cu, Sn, Fe, Zn, Ni or Mn.

4. A compound of the formula I according to claim 2, in which:
A₁ and A₂ together are CO(X)ₙ-C₁-C₃alkylene-(X)ₙCO, or CO(X)ₙ-C₁-C₃alkylene-(X)ₙCO which is substituted by C₁-C₄alkyl, COOH, COOCH₃, phenyl or the group CO-N(CH₃)₂;
X is oxygen;
X₁, X₂, and X₃ independently of one another are hydrogen, fluorine or methyl;
n is 0 or 1.

5. A compound of the formula I according to claim 3, in which:
A₁ and A₂ independently of one another are CO-C₁-C₂alkyl, COO-C₁-C₂alkyl, CO-CF₃ or CO-N(C₁-C₂alkyl)₂;
X₁, X₂, and X₃ independently of one another are hydrogen, fluorine or methyl.

6. A compound of the formula I according to claim 4, in which:
A₁ and A₂ together are CO-C₁-C₃alkylene-CO, or CO-C₁-C₃alkylene-CO which is substituted by C₁-C₂alkyl, COOH or phenyl;
X₁, X₂, and X₃ independently of one another are hydrogen or fluorine.

7. A compound of the formula I according to claim 5, in which:
A₁ and A₂ independently of one another are CO-C₁-C₂alkyl, COO-C₁-C₂alkyl or CO-N(CH₃)₂;
X₁, X₂, and X₃ independently of one another are hydrogen or fluorine.

8. A compound of the formula I according to claim 6, in which:
A₁ and A₂ together are CO-C₁-C₃alkylene-CO, or CO-C₁-C₃alkylene-CO which is substituted by methyl;
X₁, X₂ and X₃ independently of one another are hydrogen or fluorine.

9. A compound of the formula I according to claim 3, in which:
A₁ and A₂ independently of one another are CO-CH₃, COOC₁-C₄alkyl, CN or CON(CH₂C≡CH)₂;
X₁, X₂, and X₃ independently of one another are hydrogen or fluorine.

10. A compound of the formula I according to claim 2, in which:
A₁ and A₂ together are CO-N(R')-CO-N(R')-CO;
R' is hydrogen or C₁-C₃alkyl;
X₁, X₂ and X₃ independently of one another are hydrogen, fluorine or methyl.

11. A compound of the formula I according to claim 10, in which:
A₁ and A₂ together are CO-N(CH₃)-CO-N(CH₃)-CO;
X₁, X₂ and X₃ independently of one another are hydrogen or fluorine.

12. A compound of the formula I according to claim 1, selected from the group consisting of
2-(benzo-1,2,3-thiadiazole-7-carbonyl)-3-hydroxy-cyclohex-2-enone;
2-(benzo-1,2,3-thiadiazole-7-carbonyl)-3-hydroxy-5,5-dimethyl-cyclohex-2-enone;
2-(benzo-1,2,3-thiadiazole-7-carbonyl)-3-hydroxy-5-methyl-cyclohex-2-enone;
2-(benzo-1,2,3-thiadiazole-7-carbonyl)-3-hydroxy-5-isopropyl-cyclohex-2-enone;
2-(benzo-1,2,3-thiadiazole-7-carbonyl)-3-hydroxy-5-phenyl-cyclohex-2-enone;
2-(6-fluoro-benzo-1,2,3-thiadiazole-7-carbonyl)-3-hydroxy-5,5-dimethyl-cyclohex-2-enone;
2-(4-fluoro-benzo-1,2,3-thiadiazole-7-carbonyl)-3-hydroxy-cyclohex-2-enone;
5-(benzo-1,2,3-thiadiazole-7-carbonyl)-1,3-dimethyl-2,4,6-1H,3H,5H-pyrimidinetrione and
diethyl (benzo-1,2,3-thiadiazole-7-carbonyl)malonate.

13. A process for the preparation of a compound according to claim 1 of the formula I, wherein:
a) methylene compounds of the formula II
A₁-CH₂-A₂ (II)
are acylated with an activated acid derivative of the formula III 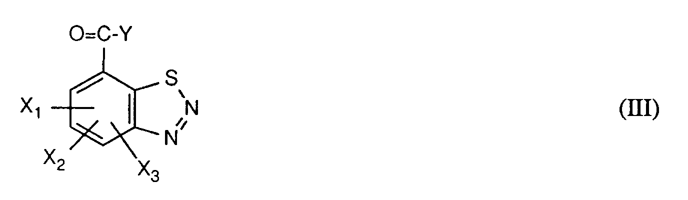 in the presence of a base with or without an addition of metal salts, for example MgCl₂ or MgO, or of a Lewis acid in inert solvents at temperatures of from -30°C to 180°C, where Y is halogen, cyano or the radical 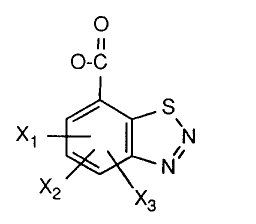 and A₁, A₂, X₁, X₂ and X₃ are as defined in formula I; or
b) the enol form of methylene compounds of the formula IIa 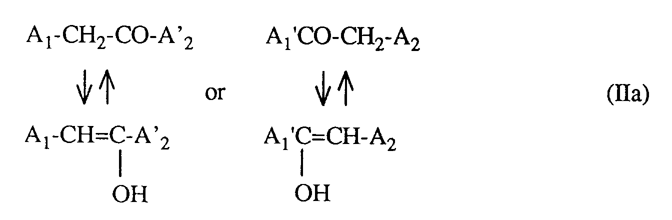 is acylated on the oxygen atom with a compound of the formula III 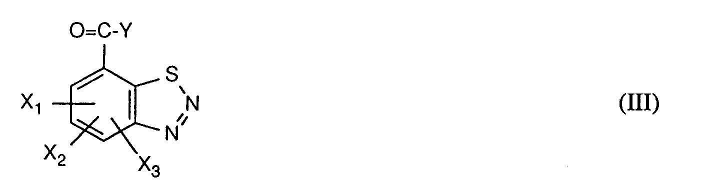 in the presence of a base in an inert solvent at temperatures of from -30°C to 160°C to obtain intermediates of the formula IV 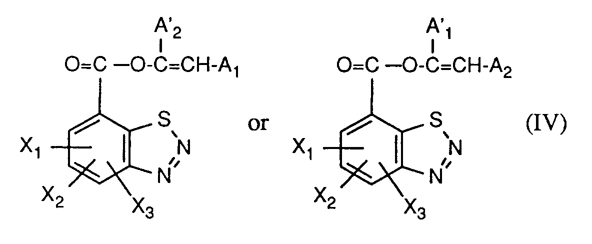 where A₁' and A₂', respectively, are defined as follows:
C₁-C₄alkyl, O-C₁-C₄alkyl, CF₃, N(R)₂, or (X)ₙ-C₁-C₃alkylene-(X)ₙCO which is unsubstituted or substituted by C₁-C₄alkyl, COOR, CON(R)₂, cyano or phenyl, in which the phenyl ring, in turn, can be substituted by halogen, methyl, trifluoromethyl, methoxy, nitro or cyano; and N(R)-CO-N(R)-CO, in which R is hydrogen, C₁-C₆alkyl, C₃-C₆alkenyl or C₃-C₆alkynyl;
and these products are rearranged with a base with or without an addition of cyanide compounds or of a Lewis acid, for example AlCl₃, as catalyst in inert solvents at temperatures of from -30°C to 160°C, in particular 0°C to 120°C, to give compounds of the formula Ia 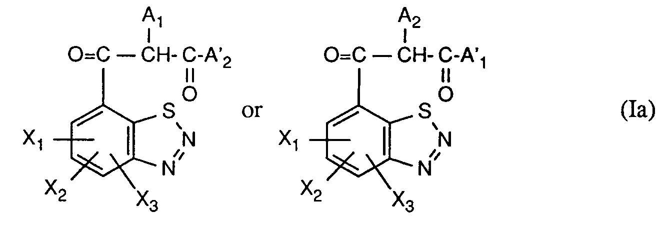 where the compounds of the formula I and Ia can also exist in the enol forms of the formula Ia' 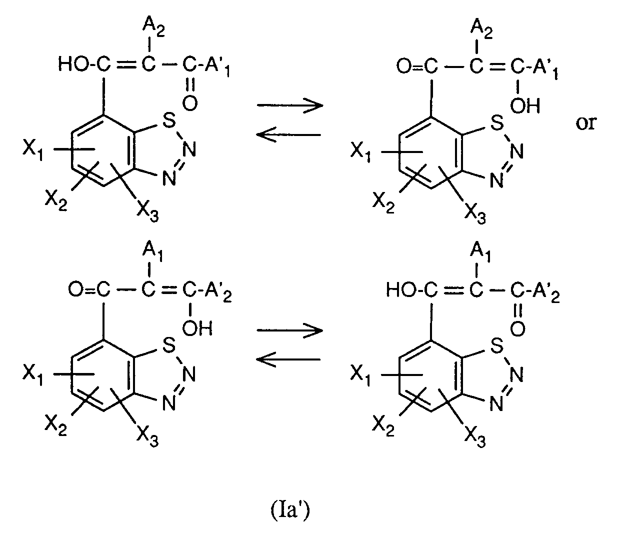 and where X₁, X₂, X₃, A₁, A₂, A₁' and A₂' are as defined in formula I, II and IV.

14. A composition for controlling or preventing infestation with noxious microorganisms, which comprises, as active component, at least one compound of the formula I according to claim 1, together with a suitable carrier material.

15. A composition according to claim 14, which comprises, as active component, at least one compound of the formula I according to claim 2.

16. A composition according to claim 14, which comprises, as active component, at least one compound of the formula I according to claim 3.

17. A composition according to claim 14, which comprises, as active component, at least one compound of the formula I according to one of claims 4 to 11.

18. A composition according to claim 14, which comprises, as active component, at least one compound of the formula I according to claim 12.

19. A method for controlling or preventing infestation of crop plants by phytopathogenic microorganisms, which comprises applying, as active ingredient, a compound of the formula I according to claim 1 to the plant, parts of the plant or its locus.

20. A process according to claim 19, wherein, as active ingredient, a compound according to one of claims 2 to 11 is applied.

21. A process according to claim 19, wherein, as active ingredient, a compound according to claim 12 is applied.

22. A process according to claim 19, wherein phytopathogenic fungi are controlled.

23. The use of a compound of the formula I according to claim 1 for controlling or preventing infestation of crop plants with phytopathogenic microorganisms, which comprises applying, as active ingredient, a compound of the formula I to the plant, parts of the plant or its locus.

24. A process for the preparation of an agrochemical composition according to claim 14, which comprises intimately mixing the components.

## Claims (Claims for the following Contracting State(s): ES)

1. A process for the preparation of a compound of the formula I 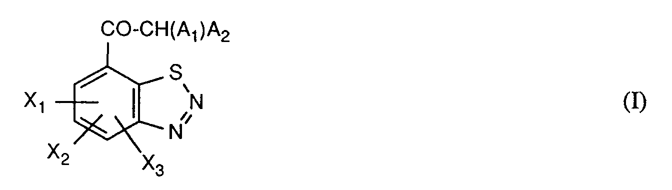 in which:
A₁ and A₂ independently of one another are CO-C₁-C₄alkyl, COO-C₁-C₄alkyl, CO-CF₃, CO-N(R)₂ or cyano;
A₁ and A₂ together are CO(X)ₙ-C₁-C₃alkylene-(X)ₙCO, CO(X)ₙ-C₁-C₃alkylene-(X)ₙCO which is substituted by C₁-C₄alkyl, COOR, CON(R)₂, cyano or phenyl, it being possible for the phenyl ring, in turn, to be substituted by halogen, methyl, trifluoromethyl, methoxy, nitro or cyano; CO-N(R)-CO-N(R)-CO;
R is hydrogen, C₁-C₆alkyl, C₃-C₆alkenyl or C₃-C₆alkynyl;
X is oxygen, sulfur or N(CH₃);
X₁, X₂ and X₃ independently of one another are hydrogen, halogen, methyl, methylthio, methoxy or nitro;
n is 0 or 1;
including the salts of the compound of the formula I with agriculturally acceptable organic or inorganic bases, and including the metal complexes, wherein:
a) a methylene compound of the formula II
A₁-CH₂-A₂ (II)
is acylated with an activated acid derivative of the formula III 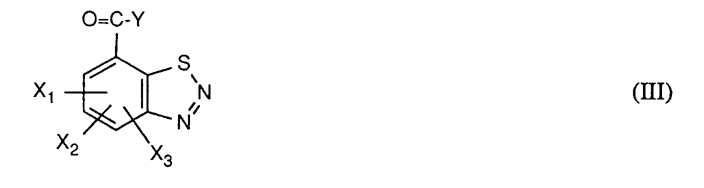 in the presence of a base with or without an addition of metal salts, for example MgCl₂ or MgO, or of a Lewis acid in inert solvents at temperatures of from -30°C to 180°C, where Y is halogen, cyano or the radical 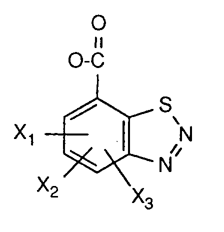 and A₁, A₂, X₁, X₂ and X₃ are as defined in formula I; or
b) the enol form of methylene compounds of the formula IIa 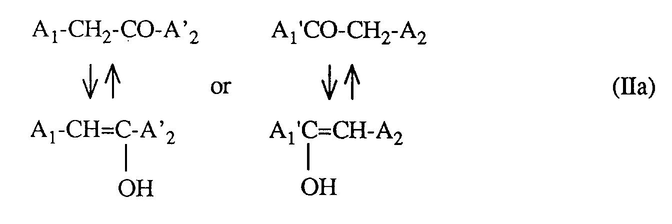 is acylated on the oxygen atom with a compound of the formula III 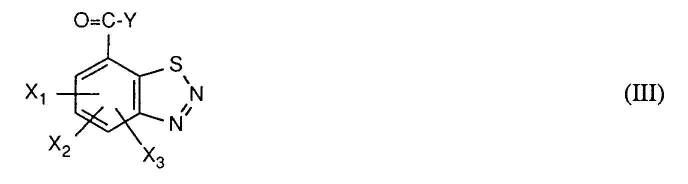 in the presence of a base in an inert solvent at temperatures of from -30°C to 160°C, to obtain intermediates of the formula IV 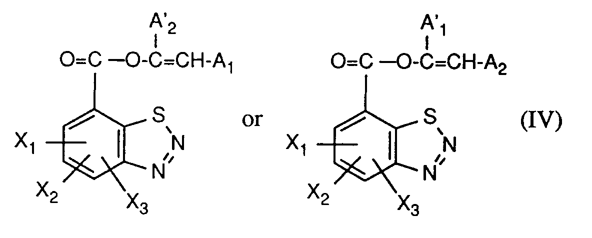 where A₁' and A₂', respectively, are defined as follows:
C₁-C₄alkyl, O-C₁-C₄alkyl, CF₃, N(R)₂, or (X)ₙ-C₁-C₃alkylene-(X)ₙCO which is unsubstituted or substituted by C₁-C₄alkyl, COOR, CON(R)₂, cyano or phenyl, in which the phenyl ring, in turn, can be substituted by halogen, methyl, trifluoromethyl, methoxy, nitro or cyano; and N(R)-CO-N(R)-CO, in which R is hydrogen, C₁-C₆alkyl, C₃-C₆alkenyl or C₃-C₆alkynyl;
and these products are rearranged with a base with or without an addition of cyanide compounds or of a Lewis acid, for example AlCl₃, as catalyst in inert solvents at temperatures of from -30°C to 160°C, in particular 0°C to 120°C, to give compounds of the formula Ia 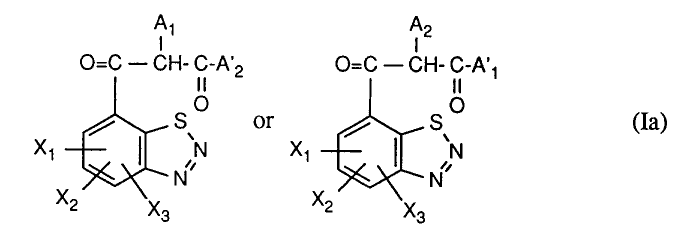 where the compounds of the formula I and Ia can also exist in the enol forms of the formula Ia' 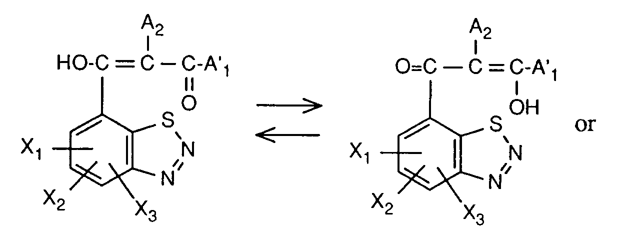 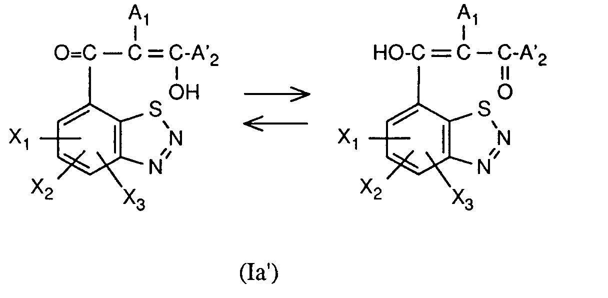 and where X₁, X₂, X₃, A₁, A₂, A₁' and A₂' are as defined in formula I, II and IV.

2. A process according to claim 1 for the preparation of a compound of the formula I in which:
A₁ and A₂ together are CO(X)ₙ-C₁-C₃alkylene-(X)ₙCO, or CO-C₁-C₃alkylene-CO which is substituted by C₁-C₄alkyl, COOH, COOCH₃, COOC₂H₅, COOCH₂-CH=CH₂, COOCH₂-C≡CH, phenyl, the group CO-N(CH₃)₂ or cyano; CO-N(R')-CO-N(R')-CO;
R' is hydrogen or C₁-C₃alkyl;
X is oxygen or sulfur;
X₁, X₂ and X₃ independently of one another are hydrogen, F, methyl, methoxy, methylthio or nitro;
n is 0 or 1;
including the salts of the compound of the formula I with organic or inorganic bases as well as the metal complexes with Mg, Ca, Ba, Sn, Fe, Zn, Cu, Ni or Mn.

3. A process according to claim 1 for the preparation of a compound of the formula I, in which:
A₁ and A₂ independently of one another are CO-C₁-C₄alkyl, COO-C₁-C₄alkyl, CO-CF₃, CO-N(R)₂ or cyano;
R is hydrogen, C₁-C₆alkyl, C₃-C₆alkenyl or C₃-C₆alkynyl;
X₁, X₂, and X₃ independently of one another are hydrogen, halogen, methyl, methylthio, methoxy or nitro;
including the salts of the compound of the formula I with agriculturally acceptable organic or inorganic bases as well as the metal complexes with Mg, Ca, Ba, Cu, Sn, Fe, Zn, Ni or Mn.

4. A process according to claim 2 for the preparation of a compound of the formula I, in which:
A₁ and A₂ together are CO(X)ₙ-C₁-C₃alkylene-(X)ₙCO, or CO(X)ₙ-C₁-C₃alkylene-(X)ₙCO which is substituted by C₁-C₄alkyl, COOH, COOCH₃, phenyl or the group CO-N(CH₃)₂;
X is oxygen;
X₁, X₂, and X₃ independently of one another are hydrogen, fluorine or methyl;
n is 0 or 1.

5. A process according to claim 3 for the preparation of a compound of the formula I in which:
A₁ and A₂ independently of one another are CO-C₁-C₂alkyl, COO-C₁-C₂alkyl, CO-CF₃ or CO-N(C₁-C₂alkyl)₂;
X₁, X₂, and X₃ independently of one another are hydrogen, fluorine or methyl.

6. A process according to claim 4 for the preparation of a compound of the formula I in which:
A₁ and A₂ together are CO-C₁-C₃alkylene-CO, or CO-C₁-C₃alkylene-CO which is substituted by C₁-C₂alkyl, COOH or phenyl;
X₁, X₂, and X₃ independently of one another are hydrogen or fluorine.

7. A process according to claim 5 for the preparation of a compound of the formula I in which:
A₁ and A₂ independently of one another are CO-C₁-C₂alkyl, COO-C₁-C₂alkyl or CO-N(CH₃)₂;
X₁, X₂, and X₃ independently of one another are hydrogen or fluorine.

8. A process according to claim 6 for the preparation of a compound of the formula I in which:
A₁ and A₂ together are CO-C₁-C₃alkylene-CO, or CO-C₁-C₃alkylene-CO which is substituted by methyl;
X₁, X₂ and X₃ independently of one another are hydrogen or fluorine.

9. A process according to claim 3 for the preparation of a compound of the formula I in which:
A₁ and A₂ independently of one another are CO-CH₃, COOC₁-C₄alkyl, CN or CON(CH₂C≡CH)₂;
X₁, X₂, and X₃ independently of one another are hydrogen or fluorine.

10. A process according to claim 2 for the preparation of a compound of the formula I in which:
A₁ and A₂ together are CO-N(R')-CO-N(R')-CO;
R' is hydrogen or C₁-C₃alkyl;
X₁, X₂ and X₃ independently of one another are hydrogen, fluorine or methyl.

11. A process according to claim 10 for the preparation of a compound of the formula I in which:
A₁ and A₂ together are CO-N(CH₃)-CO-N(CH₃)-CO;
X₁, X₂ and X₃ independently of one another are hydrogen or fluorine.

12. A process according to claim 1 for the preparation of a compound of the formula I, selected from the group consisting of
2-(benzo-1,2,3-thiadiazole-7-carbonyl)-3-hydroxy-cyclohex-2-enone;
2-(benzo-1,2,3-thiadiazole-7-carbonyl)-3-hydroxy-5,5-dimethyl-cyclohex-2-enone;
2-(benzo-1,2,3-thiadiazole-7-carbonyl)-3-hydroxy-5-methyl-cyclohex-2-enone;
2-(benzo-1,2,3-thiadiazole-7-carbonyl)-3-hydroxy-5-isopropyl-cyclohex-2-enone;
2-(benzo-1,2,3-thiadiazole-7-carbonyl)-3-hydroxy-5-phenyl-cyclohex-2-enone;
2-(6-fluoro-benzo-1,2,3-thiadiazole-7-carbonyl)-3-hydroxy-5,5-dimethyl-cyclohex-2-enone;
2-(4-fluoro-benzo-1,2,3-thiadiazole-7-carbonyl)-3-hydroxy-cyclohex-2-enone;
5-(benzo-1,2,3-thiadiazole-7-carbonyl)-1,3-dimethyl-2,4,6-1H,3H,5H-pyrimidinetrione and diethyl (benzo-1,2,3-thiadiazole-7-carbonyl)malonate.

13. A composition for controlling or preventing infestation with noxious microorganisms, which comprises, as active component, at least one compound of the formula I obtainable according to claim 1, together with a suitable carrier material.

14. A composition according to claim 13, which comprises, as active component, at least one compound of the formula I obtainable according to claim 2.

15. A composition according to claim 13, which comprises, as active component, at least one compound of the formula I obtainable according to claim 3.

16. A composition according to claim 13, which comprises, as active component, at least one compound of the formula I obtainable according to one of claims 4 to 11.

17. A composition according to claim 13, which comprises, as active component, at least one compound of the formula I obtainable according to claim 12.

18. A method for controlling or preventing infestation of crop plants by phytopathogenic microorganisms, which comprises applying, as active ingredient, a compound of the formula I obtainable according to claim 1 to the plant, parts of the plant or its locus.

19. A process according to claim 18, wherein, as active ingredient, a compound obtainable according to one of claims 2 to 11 is applied.

20. A process according to claim 18, wherein, as active ingredient, a compound obtainable according to claim 12 is applied.

21. A process according to claim 18, wherein phytopathogenic fungi are controlled.

22. The use of a compound of the formula I obtainable according to claim 1 for controlling or preventing infestation of crop plants with phytopathogenic microorganisms, which comprises applying, as active ingredient, a compound of the formula I to the plant, parts of the plant or its locus.

23. A process for the preparation of an agrochemical composition according to claim 13, which comprises intimately mixing the components.

## Revendications (Revendications pour l'(les) Etat(s) contractant(s) suivant(s): CH, DE, DK, FR, GB, IT, LI, NL)

1. Composés de formule I 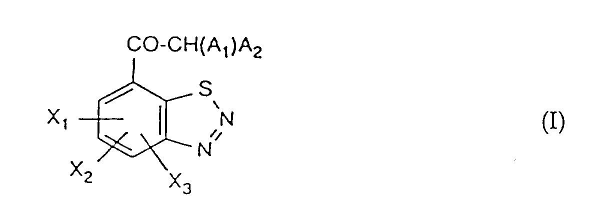
A₁ et A₂ représentent, indépendamment l'un de l'autre, un CO-alkyle en C₁-C₄, un COO-alkyle en C₁-C₄, CO-CF₃, CON(R)₂ ou le cyano;
A₁ et A₂ représentent ensemble un CO(X)ₙ-alkylène en C₁-C₃-(X)ₙCO, un CO(X)n-alkylène en C₁-C₃-(X)ₙ-CO substitué par un alkyle en C₁-C₄, COOR, CON(R)₂, le cyano ou le phényle, le noyau phénylique pouvant être, de son côté, substitué par un halogène, le méthyle, le trifluorométhyle, le méthoxy, le nitro ou le cyano; CO-N(R)-CO-N(R)-CO;
R représente l'hydrogène, un alkyle en C₁-C₆, un alcényle en C₃-C₆ ou un alcynyle en C₃-C₆;
X représente l'oxygène, le soufre ou N(CH₃);
X₁, X₂, X₃ représentent, indépendamment les uns des autres, l'hydrogène, un halogène, le méthyle, le méthylthio, le méthoxy ou le nitro;
n est égal à 0 ou 1;
incluant les sels des composés de formule I avec des bases organiques ou minérales compatibles avec les plantes, ainsi que les complexes métalliques.

2. Composés selon la revendication 1 de formule I dans laquelle :
A₁ et A₂ représentent ensemble un CO(X)ₙ-alkylène en C₁-C₃(X)nCO, un CO-alkylène en C₁-C₃-CO substitué par un alkyle en C₁-C₄, COOH, COOCH₃, COOC₂H₅, COOCH₂-CH=CH₂, COOCH₂-C--CH, le phényle, le groupe CO-N(CH₃)₂ ou le cyano; CO-N(R')-CO-N(S')-Cot
X représente l'oxygène ou le soufre;
R' l'hydrogène, un alkyle en C₁ -C₃;
X₁, X₂, X₃ représentent, indépendamment les uns des autres, l'hydrogène, F, le méthyle, le méthoxy, le méthylthio ou le nitro;
n est égal à 0 ou 1;
incluant les sels des composés de formule I avec des bases organiques ou minérales, ainsi que les complexes métalliques avec Mg, Ca, Ba, Cu, Sn, Fe, Zn, Ni, ou Mn.

3. Composés selon la revendication 1 de formule I dans laquelle
A₁ et A₂ représentent, indépendamment l'un de l'autre, un CO-alkyle en C₁-C₄, un COO-alkyle en C₁-C₄, CO-CF₃, CO-N(R) ou le cyano;
R représente l'hydrogène, un alkyle en C₁-C₆, un alcényle en C₃- C₆ ou un alcényle en C₃- C₆;
X₁, X₂, X₃ représentent, indépendamment les uns des autres, l'hydrogène, un halogène, le méthyle, le méthylthio, le méthoxy ou le nitro;
incluant les sels des composés de formule I avec les bases organiques ou minérales compatibles avec les plantes, ainsi que les complexes métalliques avec Mg, Ca, Ba, Cu, Sn, Fe, Zn, Ni ou Mn.

4. Composés de formule I selon la revendication 2, dans laquelle
A₁ et A₂ représentent ensemble un CO(X) -alkylène en C₁-C₃-(X)nCO, un CO(X)n-alkylène en Cl-C -(X)nCO substitué par un alkyle en C₁-C₄, COOH, COOCH₃, le phényle ou le groupe CO-N(CH₃)₂;
X représente, l'oxygène;
X₁, X₂, X₃ représentent, indépendamment les uns des autres, l'hydrogène, le fluor ou le méthyle; n est égal à O ou 1.

5. Composés de formule I selon la revendication 3, dans laquelle
A₁ et A₂ représentent, indépendamment l'un de l'autre, un CO-alkyle en C₁-C₂, un COO-alkyle en C₁-C₂, CO-CF₃ ou CO-N(alkyle en C₁-C₂)₂;
X₁, X₂, X₃ représentent, indépendamment les uns des autres, l'hydrogène, le fluor ou le méthyle.

6. Composés de formule I selon la revendication 4, dans laquelle
A₁ et A₂ représentent ensemble un CO-alkylène en C₁-C₃-CO, un CO-alkylène en C₁-C₃-CO substitué par un alkyle en C₁-C₂, COOH ou le phényle;
X₁, X₂, X₃ représentent, indépendamment les uns des autres, l'hydrogène ou le fluor.

7. Composés de formule I selon la revendication 5, dans laquelle
A₁ et A₂ représentent, indépendamment l'un de l'autre, un CO-alkyle en C₁-C₂, un COO-alkyle en C₁-C₂ ou CO-X(CH₃)₂;
X_{1,} X₂, X₃ représentent, indépendamment les uns des autres, l'hydrogène ou le fluor.

8. Composés de formule I selon la revendication 6, dans laquelle
A₁ et A₂ représentent ensemble un CO-alkylène en C₁-C₃-CO, un OO-alkylène en C₁-C₃-CO substitué par le méthyle;
X₁, X₂, X₃ représentent, indépendamment les uns des autres, l'hydrogène ou le fluor.

9. Composés de formule I selon la revendication 3, dans laquelle
A₁ et A₂ représentent, indépendamment l'un de l'autret CO-CH₃, COO-alkyle en C₁-C₄, CN ou CON(CH₂C=-CH)₂;
X₁, X₂, X₃ représentent, indépendamment les uns des autres, l'hydrogène ou le fluor.

10. Composés de formule I selon la revendication 2, dans laquelle
A₁ et A₂ représentent ensemble CO-N(R')-CO-N(R')-CO;
R' représente l'hydrogène, un alkyle en C₁-C₃;
X₁, X₂, X₃ représentent, indépendamment les uns des autres, l'hydrogène, le fluor ou le méthyle.

11. Composés de formule I selon la revendication 10, dans laquelle
A₁ et A₂ représentent ensemble CO-N(CH₃)-CO-N(CH₃)-CO₂.
X₁, X₂, X₃ représentent, indépendamment les uns des autres, l'hydrogène ou le fluor.

12. Un composé de formule I selon la revendication 1, choisi dans le groupe constitué par
la 2-(benzo-1,2,3-thiadiazol-7-carbonyl)-3-hydroxy-cyclohex2-énone;
la 2-(benzo-1,2,3-thiadiazol-7-carbonyl)-3-hydroxy-5,5-diméthylcyclohex- 2-énone;
la 2-(benzo-1,2,3-thiadiazol-7-carbonyl)-3-hydroxy-5-méthylcyclohex-2-énone;
la 2-(benzo-1,2,3-thiadiazol-7-carbonyl)-3-hydroxy-5-isopropylcyclohex-2-énone;
la 2-(benzo-1,2,3-thiadiazol-7-carbonyl)-3-hydroxy-5-phénylcyclohex-2-énone; l
a 2-(6-fluorobenzo-1,2,3-thiadiazol-7-carbonyl)-3-hydroxy-5,5-diméthylcyclohex-2-énone;
la 2-(4-fluorobenzo-1,2,3-thiadiazol-7-carbonyl)-3-hydroxycyclohex-2-énone;
la 5-(benzo-1,2,3-thiadiazol-7-carbonyl)-1s3-diméthyl-2,4,61H,3H,5H-pyrimidinetrione et
le (benzo-1,2,3-thiadiazol-7-carbonyl)-malonate de diéthyle.

13. Procédé pour la préparation des composés selon la revendication 1 de formule I, caractérisé
a) en ce que l'on acyle un composé méthylénique de formule II
A₁-CH₂-A₂ (II)
avec un dérivé d'un acide activé de formule III 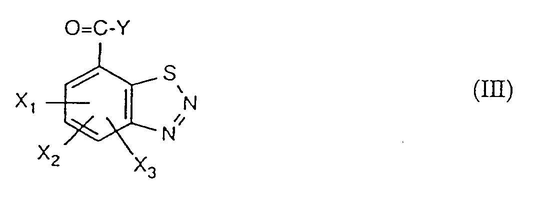 en présence d'une base ou d'un acide de Lewis, avec ou sans addition de sels métalliques tels que MgCl₂ ou MgO, dans des solvants inertes à des températures allant de -30°C à 180°C, Y représentant un halogène, le cyano ou le reste 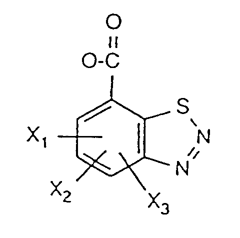 et A₁, A₂, X₁, X₂ et X₃ ont les significations indiquées pour la formule I ou
b) en ce que, partant des composés méthyléniques de formule IIa 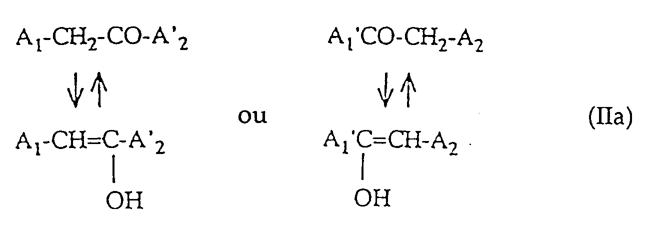 on acyle la forme énolique sur l'atome d'oxygène avec un composé de formule III 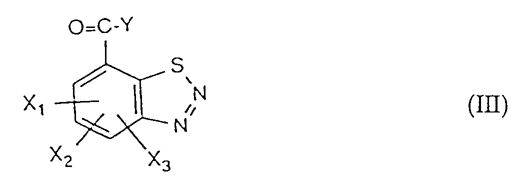 en présence d'une base dans un solvant inerte à des températures allant de -30°C à 160°C, en passant par les produits intermédiaires de formule IU 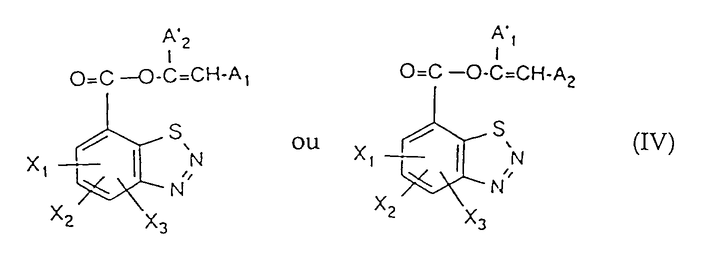 A'₁ où A'₂ ayant les significations suivantes:
un alkyle en C₁-C₄, un O-alkyle en C₁-C₄, CF₃, N(R)₂, un (X) -alkylène en C₁-C₃-(X)nCO non substitué ou substitué par un alkyle en C₁-C₄, COOR, CON(K)₂, le cyano ou le phényle, le noyau phénylique pouvant étre, de son côté, substitué par un halogène, le méthyle, le trifluorométhyle, le méthoxy, le nitro ou le cyano et N(R)-CO-N(R)-CO R représentant l'hydrogène, un alkyle en C₁-C₆, un alcényle en C₃-C₆ ou un alcynyle en C₃-C₆; et ceux-ci étant transposés avec une base avec ou sans addition de composés cyanures ou d'un acide de Lewis tel que par exemple AlCl, comme catalyseur dans des solvants inertes à des températures allant de -30°C à 160°C, en particulier allant de 0°C à 120°C, en composés de formule Ia 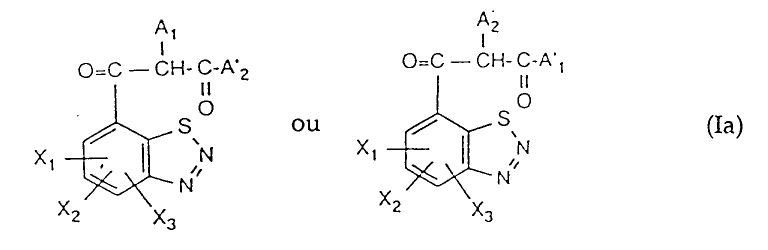 les composés de formules I et Ia pouvant également se présenter sous les formes énoliques de formules Ia' 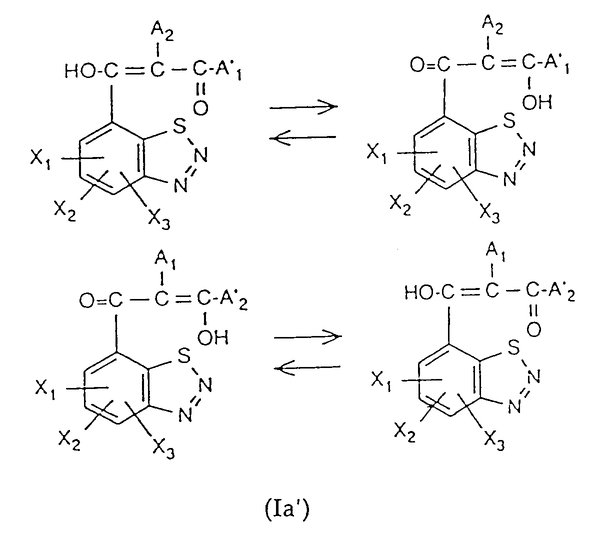 et X₁, X₂, X₃, A₁, A₂, A'₁ et A'₂ ayant les significations indiquées pour les formules I, II et IV.

14. Composition pour lutter contre ou prévenir une attaque par les mieroorganismes nuisibles, caractérisée en ce qu'elle contient, à titre de composant actif, au moins un composé de formule I selon la revendication 1, associé à un support approprié.

15. Composition selon la revendication 14, caractérisée en ce qu'elle contient, à titre de composant actif, au moins un composé de formule I selon la revendication 2.

16. Composition selon la revendication 14, caractérisée en ce qu'elle contient, à titre de composant actif, au moins un composé de formule I selon la revendication 3.

17. Composition selon la revendication 14, caractérisée en ce qu'elle contient, à titre de composant actif, au moins un composé de formule I selon l'une des revendications 4 à 11.

18. Composition selon la revendication 14, caractérisée en ce qu'elle contient, à titre de composant actif, au moins un composé de formule I selon la revendication 12.

19. Procédé pour lutter contre ou prévenir une attaque des microorganismes plantes cultivées par les microorganismes phytopathogènes, caractérisé en ce que l'on applique, à titre de substance active, un composé de formule I selon la revendication 1 sur la plante, sur les parties de la plante ou sur l'endroit où elle se trouve.

20. Procédé selon la revendication 19, caractérisé en ce que l'on applique, à titre de substance active, un composé selon l'une des revendications 2 à 11.

21. Procédé selon la revendication 19, caractérisé en ce que l'on applique, à titre de substance active, un composé selon la revendication 12.

22. Procédé selon la revendication 19, caractérisé en ce que l'on combat les champignons phytopathogènes.

23. Utilisation des composés de formule I selon la revendication 1 pour lutter contre ou prévenir une attaque des plantes cultivées par les mi croorganismes phytopathogènes, caractérisée en ce que l'on applique, à titre de substance active, un composé de formule I sur la plante, sur les parties de la plante ou sur l'endroit où elle se trouve,

24. Procédé pour la préparation d'une composition agro-chimique selon la revendication 14, caractérisé en ce que l'on mélange intimement les composants.

## Revendications (Revendications pour l'(les) Etat(s) contractant(s) suivant(s): ES)

1. Procédé pour la préparation des composés de formule I 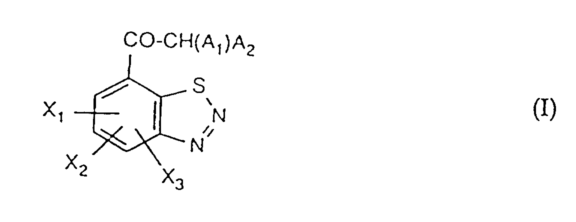 dans laquelle
A₁ et A₂ représentent, indépendamment l'un de l'autre, un CO-alkyle en C₁-C₄, un COO-alkyle en C₁-C₄, CO-CF₃, CON(R)₂ ou le cyano;
A₁ et A₂ représentent ensemble un CO(X)n-alkylène en Cl-C₃-(X)nCO, un CO(X)n-alkylène en Cl-C₃-(X)n-CO substitué par un alkyle en C₁-C₄, COOR, CON(R)₂, le cyano ou le phényleb le noyau phénylique pouvant être, de son côté, substitué par un halogène, le méthyle, le trifluorométhyle, le méthoxy, le nitro ou le cyano; CO-N(R)-CO-N(R)-CO;
R représente l'hydrogène, un alkyle en C₁-C₆, un alcényle en C₃-C₆ ou un alcynyle en C₃-C₆;
X représente l'oxygène, Le soufre ou N(CH₃); X₁, X₂, X₃ représentent, indépendamment les uns des autres, l'hydrogène, un halogène, le méthyle, le méthylthio, le méthoxy ou le nitro;; n est égal à O ou 1;
incluant les sels des composés de formule I avec des bases organiques ou minérales compatibles avec les plantes, ainsi que les complexes métalliques. caractérisé
a) en ce que l'on acyle un composé méthyléniq ue de formule II
A₁-CH₂-A₂ (II)
avec un dérivé d'un acide activé de formule III 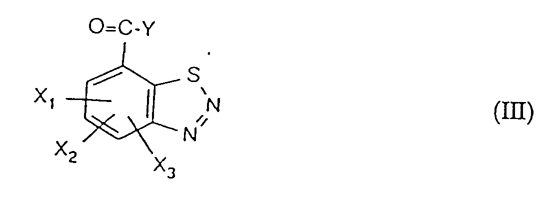 en présence dtune base ou d'un acide de Lewis,avec ou sans addition de sels métalliques tels que MgCl₂ ou MgO, dans des solvants inertes à des températures allant de -30°t à 180°C, allant dc 0°C a 190°C, Y représentant un halogène, le cyano ou le reste 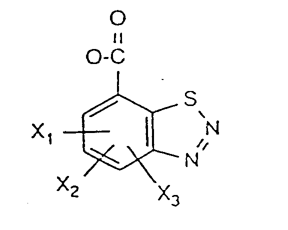 et A₁, A₂, X₁₂ X₂ et X₃ ont les significations indiquées pour la formule I ou
b) en ce que l'on acyle la forme énolique des composés méthyléniques de formule IIa 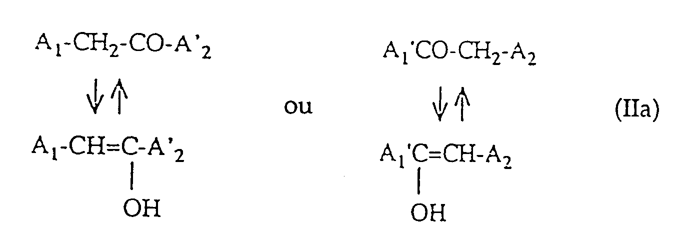 l'atome d'oxygène avec un composé de formule III 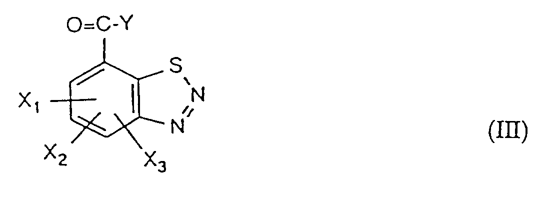 en présence d'une base dans un solvant inerte à des températures allant de -30°C à 160°C, en passant par les produits intermédiaires de formule IV; 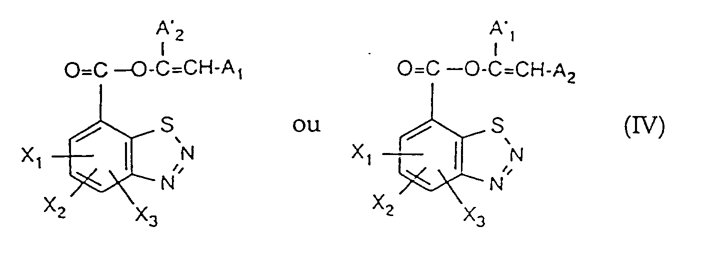 A'₁ ou A'₂ ayant les significations suivantes:
un allçyle en C₁-C₄, un O-alkyle en C₁-C₄, CF₃, N(R)₂, un (X)n-alkylène en C₁-C₃-(X)nCO non substitué ou substitué par un alkyle en C₁-C₄, COOR, CON₁9)₂, le cyano ou le phényles le noyau phénylique pouvant être, de son caté, substitué par un halogène, le méthyle, le trifluorométhyle, le méthoxy, le nitro ou le cyano et N(R)-CO-N(R)-C0₃ R représentant l'hydrogène, un alkyle en C₁-C₆, un alcényle en C₃-C₆ ou un alcynyle en C₃-C₆; et ceux-ci étant transsosés avec une base avec ou sans addition de composés cyanures ou d'un acide de Lewis tel que par exemple, comme catalyseur dans des solvants inertes à des températures allant de -30°C à 160°C, en particulier allant de 0°C à 120°C, en composés de formule Ia 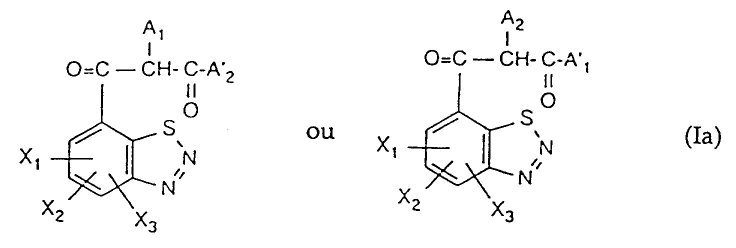 les composés de formules I et Ia pouvant également se présenter sous les formes énoliques de formules (Ia') 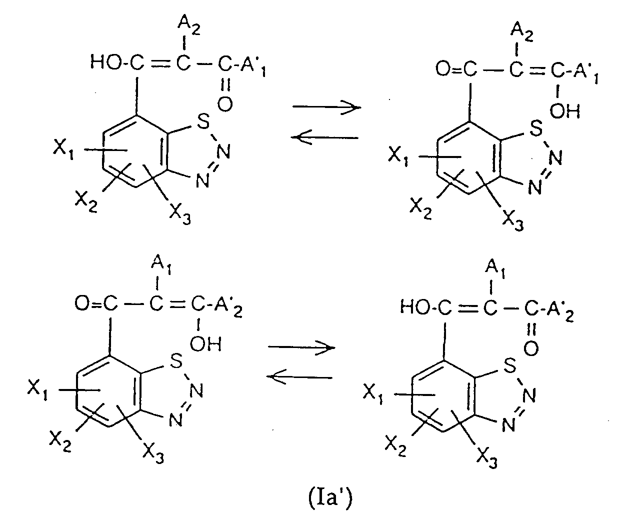 et X₁, X₂, X₃, A₁, A₂, A'₁ et A'₂ ayant les significations indiquées pour les formules I, II et IV.

2. Procédé selon la revendication 1 pour la préparation des composés de formule I où
A₁ et A₂ représentent ensemble un CO(X)n-alkylène en C₁-C₃(X)nCO, un CO-alkylène en C₁-C₃-CO substitué par un alkyle en C₁-C₄, COOH, COOCH₃, COOC₂H₅, COOCH₂-CH≡CH₂, COOCH₂-C--CH, le phényle, le groupe CO-N(CH₃)₂ ou le cyano; CO-N(R')-CO-N(XI)-CO;
X représente l'oxygène ou le soufre;
R' l'hydrogène, un alkyle en C₁-C₃;
X₁, X₂, X₃ représentent, indépendamment les uns des autres, l'hydrogène, F, le méthyle, le méthoxy, le méthylthio ou le nitro; n est égal à O ou 1; incluant les sels des composés de formule I avec des bases organiques ou minérales, ainsi que les complexes métalliques avec Mg, Ca, Ba, Cu, Sn, Fe, Zn, Ni, ou Mn.

3. Procédé selon la revendication 1 pour la préparation des composés de formule I où
A₁ et A₂ représentent, indépendamment l'un de l'autre, un CO-alkyle en C₁-C₄, un COO-alkyle en C₁-C₄, CO-CR₃, CO-N(R)₂ ou le cyano;
R représente l'hydrogène, un alkyle en C₁-C₆, un alcényle en C₃-C₆ ou un alcynyle en C₃-C₆;
X₁, X₂, X₃ représentent, indépendamment les uns des autres, l'hydrogène, un halogène, le méthyle, Re méthylthio, le méthoxy ou le nitro; incluant les sels des composés de formule I avec les bases organiques ou minérales compatibles avec les plantes, ainsi que les complexes métalliques avec Mg, Ca, Ba, Cu, Sn, Fe, Zn, Ni ou Mn.

4. Procédé selon la revendication 2 pour la préparation deS composés de formule I où
A₁ et A₂ représentent ensemble un CO(X) -alkylène en C₁-C₃-(X)nCO, un CO(X) -alkylène en C₁-C₃-(X)nCO substitué par un alkyle en C₁-C₄, COOH, COOCH₃, le phényle ou le groupe CO-N(CH₃)₂;
X représente l'oxygène;
X₁, X₂, X₃ représentent, indépendamment les uns des autres, l'hydrogène, le fluor ou le méthyle; n est égal à 0 ou 1.

5. Procédé selon la revendication 3 pour la préparation des composés de formule I où
A₁ et A₂ représentent, indépendamment l'un de l'autre, un CO-alkyle en C₁-C₂, un COO-alkyle en C₁-C₂, CO-CF₃ ou un CO-N(alkyle en C₁ - C₂)₂;
X₁, X₂, X₃ représentent, indépendamment les uns des autres, l'hydrogène, le fluor ou le méthyle.

6. Procédé selon la revendication 4 pour la préparation des composés de formule I où
A₁ et A₂ représentent ensemble un CO-alkylène en C₁-C₃-CO, un CO-alkylène en C₁-C₃-CO substitué par un alkyle en C₁-C₂, COOH ou le phényle;
X₁, X₂, X₃ représentent, indépendamment les uns des autres, l'hydrogène ou le fluor.

7. Procédé selon la revendication 5 pour la préparation des composés de formule I où
A₁ et A₂ représentent, indépendamment l'un de l'autre, un CO-alkyle en C₁-C₂, un COO-alkyle en C₁-C₂ ou CO-N(CH₃)₂; X₁, X₂, X₃ représentent, indépendamment les uns des autres, l'hydrogène ou le fluor.

8. Procédé selon la revendication 6 pour la préparation des composés de formule I où
A₁ et A₂ représentent ensemble un CO-alkylène en C₁-C₃-CO, un CO-alkylène en C₁-C₃-CO substitué par le méthyle;
X₁, X₂, X₃ représentent, indépendamment les uns des autres, l'hydrogène ou le fluor.

9. Procédé selon la revendication 3 pour la préparation des composés de formule I où
A₁ et A₂ représentent, indépendamment l'un de l'autre, CO-CH₃, COO-alkyle en C₁-C₄, CN ou C(N(CH₂C≡CH)₂;
X₁, X₂, X₃ représentent, indépendamment les uns des autres, l'hydrogène ou le fluor.

10. Procédé selon la revendication 2 pour la préparation des composés de formule I où
A₁ et A₂ représentent ensemble CO-N(R')-CO-N(R')-CO;
R₁ représente l'hydrogène, un alkyle en C₁-C₃;
X₁, X₂, X₃ représentent, indépendamment les uns des autres, l'hydrogène, le fluor ou le méthyle.

11. Procédé selon la revendication 10 pour la préparation des composés de formule I où
A₁ et A₂ représentent ensemble CO-N(CH₃)-CO-N(CH₃)-CO;
X₁, X₂, X₃ représentent, indépendamment les uns des autres, l'hydrogène ou le fluor.

12. Procédé selon la revendication 1 pour la préparation d'un composé de formule m choisi dans le groupe constitué par
la 2-(benzo-1,2,3-thiadiazol-7-carbonyl)-3-hydroXy-cyclohex-2-énone;
la 2-(benzo-1,2,3-thiadiazol-7-carbonyl)-3-hydroXy-5,5-diméthylcyclohex-2-énone;
la 2-(benzo-1,2,3-thiadiazol-7-carbonyl)-3-hydroxy-5-mèthylcyclohex-2-énone;
la 2-(benzo-1,2,3-thiadiazol-7-carbonyl)-3-hydroXy-5-isopropylcyclohex2-énone;
la 2-(benzo-1,2,3-thiadiazol-7-carbonyl)-3-hydroXy-5-phénylcyclohex-2-énone;
la 2-(6-fluorobenzo-1,2,3-thiadiazol-7-carbonyl)-3-hydroxy5,5-diméthylcyclohex-2-énone;
la 2-(4-fluorobenzo-1,2,3-thiadiazol-7-carbonyl)-3-hydroxycyclohex-2-énone;
la 5-(benzo-1,2,3-thiadiazol-7-carbonyl)-1,3-diméthyl-2,4,6-1H,3H,5H-pyrimidinetrione et
le (benzo-1,2,3-thiadiazol-7-carbonyl)-malonate de diéthyle.

13. Composition pour lutter contre ou prévenir une attaque par les microorganismes nuisibles, caractérisée en ce qu'elle contient, à titre de composant actif, au moins un composé de formule I obtenu selon la revendication 1, associé à un support approprié.

14. Composition selon la revendication 13, caractérisée en ce qu'elle contient, à titre de composant actif, au moins un composé de formule I obtenu selon la revendication 2.

15. Composition selon la revendication 13, caractérisée en ce qu'elle contient, à titre de composant actif, au moins un composé de form ule I obtenu selon la revendication 3.

16. Composition selon la revendication 13, caractérisée en ce qu'elle contient, à titre de composant actif, au moins un composé de formule I obtenu selon l'une des revendications 4 à 11.

17. Composition selon la revendication 13, caractérisée en ce qu'elle contient, à titre de composant actif, au moins un composé de formule I obtenu selon la revendication 12.

18. Procédé pour lutter contre ou prévenir une attaque des plantes cultivées par les microorganismes phytopathogènes, caractérisé en ce que l'on applique, à titre de substance active, un composé de formule I obtenu selon la revendication 1 sur la plante, sur les parties de la plante ou sur l'endroit où elle se trouve.

19. Procédé selon la revendication 18, caractérisé en ce que l'on applique, à titre de substance active, un composé obtenu selon l'une des revendications 2 à 1 1.

20. Procédé selon la revendication 18, caractérisé en ce que l'on applique, à titre de substance active, un composé obtenu selon la revendication 12.

21. Procédé selon la revendication 18, caractérisé en ce que l'on combat les champignons phytopathogènes.

22. Utilisation des composés de formule I obtenus selon la revendication 1 pour lutter contre ou prévenir une attaque des plantes cultivées par les microorganismes phytopathogènes, caractérisée en ce que l'on applique, à titre de substance active, un composé de formule I sur la plante, sur les parties de la plante ou sur l'endroit où elle se trouve.

23. Procédé pour la préparation d'une composition agro-chimique selon la revendication 13, caractérisé en ce que l'on mélange intimement les composants.
